# EUROPEAN PATENT APPLICATION

(11) **EP 4 394 030 A1**
(43) Date of publication of application: **03.07.2024**
(21) Application number: 22860648.9
(22) Date of filing: 26.08.2022
(51) Int. Cl.: C12N 5/071, C12N 5/078, C12N 5/0783

(54) **INDUCED TOTIPOTENT STEM CELLS AND PREPARATION METHOD THEREFOR**

(30) Priority: 26.08.2021 CN 202110989429; 09.06.2022 CN 202210654031
(71) Applicant: Tsinghua University, Haidian District, Beijing 100084 (CN)
(72) Inventor: DING, Sheng, Beijing 100084 (CN); LIU, Kang, Beijing 100084 (CN); HU, Yanyan, Beijing 100084 (CN); YANG, Yuanyuan, Beijing 100084 (CN); TAN, Pengcheng, Beijing 100084 (CN); MA, Tianhua, Beijing 100084 (CN)
(74) Representative: Cabinet Becker et Associés
(86) International application number: PCT/CN2022/115235
(87) International publication number: WO 2023/025302

(57) **Abstract**

The invention relates to a combination of small molecule reprogramming agents for inducing the generation of totipotent stem cells, a method for preparing the induced totipotent stem cells and the induced totipotent stem cells generated therefrom.

## Description

### TECHNICAL FIELD

The invention relates to a combination of small molecule reprogramming agents for inducing the generation of totipotent stem cells, a method for generating induced totipotent stem cells (ciTotiSC), and the induced totipotent stem cells generated by induction.

### BACKGROUND OF THE INVENTION

Embryonic development of mammals starts from the zygote formed by the binding of oocyte and sperm, followed by developing into stages such as 2-cell stage, 4-cell stage, 8-cell stage and morula through embryo cleavage.

For example in mice, only the zygote and 2-cell-stage blastomere have intra-embryonic and extra-embryonic bidirectional developmental potential and the capacity of forming an entire organism, i.e., totipotency, and they are called totipotent cells.

As the embryo development proceeds, totipotent cells differentiate into two cell types, the inner cell mass (ICM) and the trophoblast cells (TE). The embryo at this stage is called a blastocyst.

TEs are located outside the embryo at the blastocyst stage and can develop into extra-embryonic parts such as the placenta. TEs play an important role in further differentiation of the ICM. The cells of ICM are further differentiated into primitive endoderm (PE) and epiblast (EPI). Among them, the PE further develops into extra-embryonic tissue -- visceral yolk sac, while the EPI further develops into various tissues and organs of the fetus and eventually develops into a complete fetus.

Since EPI can only develop into the embryonic parts and loses the capacity to develop into extra-embryonic parts, it possesses pluripotency instead of the totipotency of embryonic/extra-embryonic bidirectional development, and thus it is called pluripotent cells. Embryonic stem cells (ESCs) from cultured ICM *in vitro* are pluripotent as well, and can differentiate into all embryonic tissues and proliferate unlimitedly *in vitro.*

In 1981, Martin Evans and Matthew Kaufman from the University of Cambridge successfully isolated the first line of pluripotent mouse embryonic stem cells (mESC) from mouse embryos for the first time, triggering a boom in embryonic stem cell research.

In 2006, Shinya Yamanaka reprogrammed previously highly-differentiated somatic cells into induced pluripotent stem cell (iPSC) of pluripotency by transfecting a combination of 4 transcription factors (Oct4, Sox2, Klf4 and c-Myc) into somatic cells with viral vectors, and thus is awarded with the Nobel Prize in 2012.

In the 40 years since the establishment of pluripotent mESCs, all cultured mESCs were in a pluripotent state. Attempts have been made all the time to obtain stem cells with higher developmental potential *in vitro.* To date, however, the *in vitro* induction and prolonged culture of totipotent stem cells that resemble *in vivo* totipotent embryonic cells both at the molecular level and function remains a great challenge.

SHEN Hui *et al.* (Shen H, Yang M, Li S, et al. Mouse totipotent stem cells captured and maintained through spliceosomal repression [J]. Cell, 2021, 184(11): 2843-2859. e20.) reported that spliceosomal repression in mouse ESCs drives a pluripotent-to-totipotent state transition. Using the splicing inhibitor pladienolide B, this study achieves a new cell type which is comparable at transcriptome levels with 2- and 4-cell blastomeres, and is called totipotent blastomere-like cells (TBLCs). Mouse chimeric assays combined with RNA sequencing (RNA-seq) demonstrate that the TBLCs have a robust bidirectional developmental capability to generate multiple intra-embryonic and extra-embryonic cell lines. Mechanically, spliceosomal repression causes widespread splicing inhibition of pluripotent genes, whereas totipotent genes, which contain few short introns, are efficiently spliced and transcriptionally activated. However, the splicing inhibition technique for converting pluripotent stem cells into stem cells having certain totipotency in this study requires multiple subculturing and takes a considerable time period. Moreover, by analyzing the scRNA-seq data of TBLCs and normal mouse embryos obtained in this study, the inventors found that the TBLCs are close to the cells of the post-implantation embryo at a later development stage rather than to the totipotent zygote and 2-cell-stage blastomeres. Thus they cannot be deemed as totipotent cells. More importantly, the TBLCs have not been shown to have the capacity to independently develop into mouse embryos and thus generate an entire organism. They do not meet the strict definition of totipotent cells (which can be independently developed into an entire organism) and thus cannot be defined as totipotent stem cells.

It is commonly recognized in the art that a cell with totipotency should meet one or more of the following, preferably two, and more preferably all three: 1) the cells are similar to totipotent embryonic cells, i.e., zygotes and two-cell blastomeres, in terms of transcription level; 2) further, the cells have the potential of bidirectional development to both intra-embryonic and extra-embryonic cell types; 3) more further and most strictly, one cell can develop into a complete embryo or a living individual.

There is still a need in the art to induce more desirable totipotent stem cells, especially in a more rapid and efficient way. The resulting induced totipotent stem cells meet the above definition of totipotency.

### SUMMARY OF THE INVENTION

The inventors surprisingly found that pluripotent stem cells can be induced into totipotency stem cells (referred to herein as ciTotiSC, namely "Chemical Induced TotiPotent Stem Cell", "chemically induced totipotent stem cells", or simply "induced totipotent stem cells") by using a specific compound combination as additives for the basal medium of pluripotent stem cells, and the induction is very fast and effective.

Therefore, in one aspect, the present invention provides a composition, comprising:
(a) a RA signaling pathway activator; and
(b) one or more of a GSK-3 inhibitor, an IKK signaling pathway inhibitor, an HDAC inhibitor, a histone methyltransferase inhibitor, a Src kinase inhibitor, a cAMP activator, and a cell metabolism modulator.

In another aspect, the present invention provides a kit, comprising:
(a) a RA signaling pathway activator; and
(b) one or more of a GSK-3 inhibitor, an IKK signaling pathway inhibitor, an HDAC inhibitor, a histone methyltransferase inhibitor, a Src kinase inhibitor, a cAMP activator, and a cell metabolism modulator.

In another aspect, the present invention provides use of (a) an RA signaling pathway activator; and (b) one or more of a GSK-3 inhibitor, an IKK signaling pathway inhibitor, an HDAC inhibitor, a histone methyltransferase inhibitor, a Src kinase inhibitor, a cAMP activator, and a cell metabolism modulator in manufacturing induced totipotent stem cells.

In one or more of some embodiments of the foregoing aspects, the RA signaling pathway activator is selected from small molecules for the same pathway such as TTNPB, Tretionin/RA/ATRA, AM580, Taza, 9-cis-RA, Acitretin, CD437, Tamibarotene, Tazarotene, retinoic acid, Isotretinoin, Acitretin sodium, ch55, and AC55649, and the others.

In one or more of some embodiments of the foregoing aspects, the GSK-3 inhibitor is selected from small molecules for the same pathway such as 1-Azakenpaullone, AZD2858, CHIR99021 and AZD1080, and the others.

In one or more of some embodiments of the foregoing aspects, the IKK signaling pathway inhibitor is selected from small molecules for the same pathway such as WS6, sc-514, PF184 and IKK16, and the others.

In one or more of some embodiments of the foregoing aspects, the HDAC inhibitor is selected from the small molecules for same pathway such as Trichostatin A (TSA), Valproic acid (VPA), Vorinostat (SAHA), and Entinostat (MS-275), and the others.

In one or more of some embodiments of the foregoing aspects, the histone methyltransferase inhibitor is selected from small molecules for the same pathway such as BIX 01294, 3-deazaneplanocin A (DZNeP) HCl, A-366, UNC0638, SGC 0946, and the others.

In one or more of some embodiments of the foregoing aspects, the Src kinase inhibitor is selected from small molecules for the same pathway such as Dasatinib (BMS-354825), WH-4-023, Ponatinib (AP24534), Bosutinib (SKI-606), and the others.

In one or more of some embodiments of the foregoing aspects, the cAMP activator is selected from small molecules for the same pathway such as Colforsin (Forskolin, HL 362) and 8-Br-cAMP, and the others.

In one or more of some embodiments of the foregoing aspects, the cell metabolism modulator is selected from the cell metabolism modulators such as 2-Deoxy-D-glucose (2-DG), sodium acetate, sodium L-lactate, and D-ribose, and the others.

In another aspect, the present invention provides a culture medium, comprising the compositions described herein.

In one or more of some embodiments of the foregoing aspects, the culture medium comprises a basal culture medium.

In one or more of some embodiments of the foregoing aspects, the basal medium is selected from the common basal media such as DMEM, Knockout DMEM, RPMI 1640 and DMEM/F12, and the others.

In another aspect, the present invention provides a method of manufacturing induced totipotent stem cells, comprising culturing pluripotent stem cells in the culture medium described herein, thereby manufacturing the induced totipotent stem cells.

In one or more of some embodiments of the foregoing aspects, the pluripotent stem cells are embryonic stem cells or induced pluripotent stem cells.

In one or more of some embodiments of the foregoing aspects, the method includes reprogramming non-pluripotent cells into pluripotent stem cells.

In one or more of some embodiments of the foregoing aspects, the non-pluripotent cells are selected from somatic cells and/or adult stem cells.

In one or more of some embodiments of the foregoing aspects, the reprogramming non-pluripotent cells into pluripotent stem cells comprises expressing one or more reprogramming factors selected from the group consisting of Oct4, Sox2, Klf4 and c-Myc in the non-pluripotent cells.

In one or more of some embodiments of the foregoing aspects, the culturing pluripotent stem cells are performed for 1, 2, 3, 4, 5, 6, 7, 8, 9, 10, 11, 12, 13, 14, 15, 16, 17, 18, 19, or 20 days.

In another aspect, the present invention provides a culture, comprising the culture medium described herein and pluripotent stem cells.

In one or more of some embodiments of the foregoing aspects, the pluripotent stem cells are embryonic stem cells or induced pluripotent stem cells.

In another aspect, the present invention provides a culture, comprising the culture medium described herein and totipotent stem cells.

In one or more of some embodiments of the foregoing aspects, the totipotent stem cells are induced totipotent stem cells, preferably manufactured by methods described herein.

In another aspect, the present invention provides an induced totipotent stem cell characterized by one or more of the following:
(a) increased transcription of one or more totipotent transcription markers selected from the group consisting of MERVL, Zscan4c, Zscan4d, Zscan4f, Zfp352, Tcstv1, Tcstv3, Teme92, Gm6763;
(b) reduced transcription of one or more pluripotent transcription markers selected from the group consisting of POU5f1, ZFP42, NANOG, KLF4, ESRRB; and
(c) the ability to differentiate into extra-embryonic cell type(s);

Preferably, the induced totipotent stem cells can be manufactured by the methods described herein.

In another aspect, the present invention provides an induced totipotent stem cell, which can be manufactured by the method according to any one of the preceding claims.

In another aspect, the present invention provides an organism derived from the induced totipotent stem cells described herein, preferably wherein the organism is a rodent animal or a mammal animal.

In another aspect, the present invention provides an organoid produced from the induced totipotent stem cells described herein.

In another aspect, the present invention provides a tissue produced from the induced totipotent stem cells described herein, preferably the tissue is blood.

In another aspect, the present invention provides a differentiated cell that is differentiated from the induced totipotent stem cells described herein, preferably wherein the differentiated cell is a blood cell or an immune cell, such as a T cell or an NK cell.

### BRIEF DESCRIPTION OF THE DRAWINGS

Figure 1.1: Subculture of mouse induced totipotent stem cells (ciTotiSCs) of the present invention.
Figure 1.2: The highly expressed totipotency marker gene of the mouse induced totipotent stem cells (ciTotiSCs) of the present invention.
Figure 2.1: Specific gene set enrichment analysis (GSEA) in mouse induced totipotent stem cells (ciTotiSCs) of the present invention and mouse pluripotent embryonic stem cells (mESCs) (top panel).
   The mouse induced totipotent stem cells (ciTotiSCs) of the present invention has high expression of maternal genes, ZGA genes, totipotent genes, and low expression of pluripotent specific genes (bottom panel) compared with mouse pluripotent embryonic stem cells (mESCs), totipotent blastomere-like cells (TBLCs), totipotent-like cells (TLSCs), and expanded potential stem cells (EPSCs).

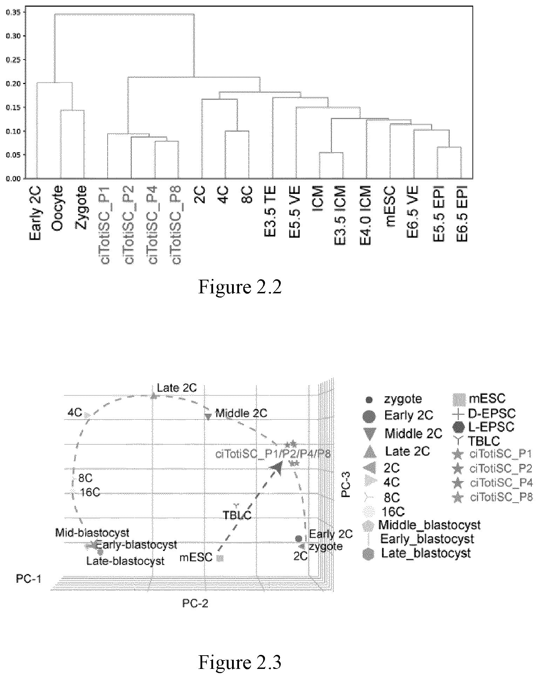

Figure 2.2: Clustering analysis at the whole transcriptome level of the mouse induced totipotent stem cells (ciTotiSCs) of the present invention and mouse pluripotent embryonic stem cells (mESCs).
Figure 2.3: Principal Component Analysis (PCA) at the whole transcriptome level of the mouse induced totipotent stem cells (ciTotiSCs) of the present invention and mouse pluripotent embryonic stem cells (mESCs).

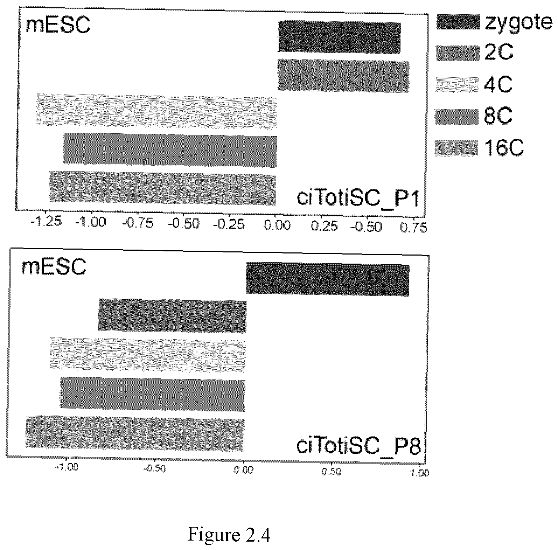

Figure 2.4: Gene set enrichment analysis (GSEA) of the mouse induced totipotent stem cells (ciTotiSCs) of the present invention, mouse pluripotent embryonic stem cells (mESCs) and totipotent blastomere-like cells (TBLCs) and different stages of embryonic development in normal mouse embryos.

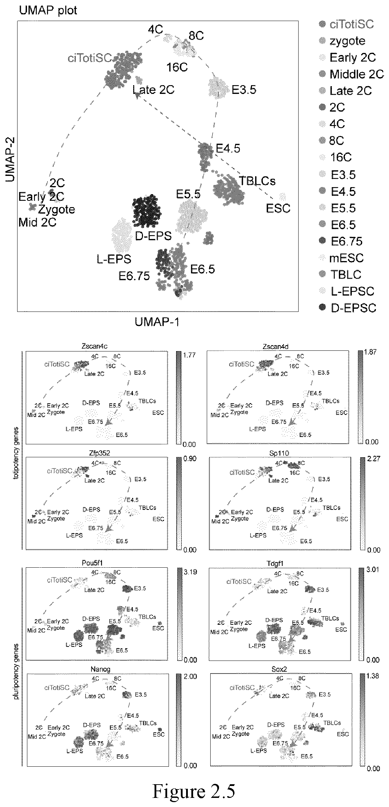

Figure 2.5: Single-cell RNA sequencing (scRNA-seq) UMAP analysis of the mouse induced totipotent stem cells (ciTotiSCs) of the present invention and mouse pluripotent embryonic stem cells (ESCs) and totipotent blastomere-like cells (TBLCs) and normal mouse embryos at various stages.

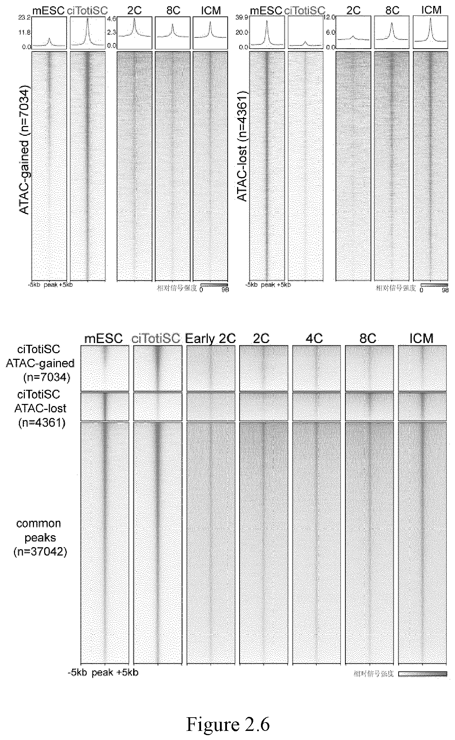

Figure 2.6: Transposase-accessible chromatin sequencing (ATAC-seq) analysis of the mouse induced totipotent stem cells (ciTotiSCs) of the present invention and mouse pluripotent embryonic stem cells (mESCs).

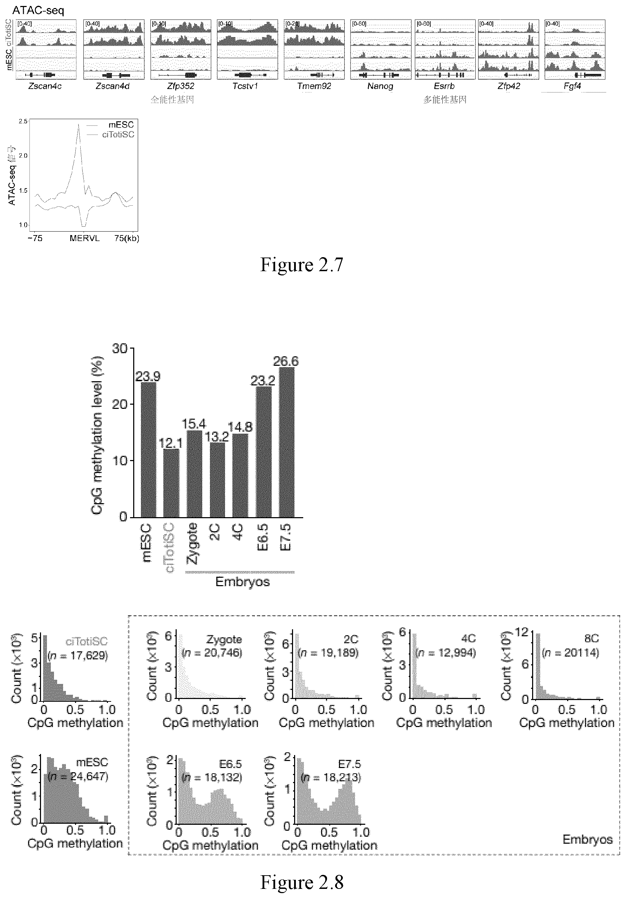

Figure 2.7: Site-specific transposase-accessible chromatin sequencing (ATAC-seq) analysis of the mouse induced totipotent stem cells (ciTotiSCs) of the present invention and mouse pluripotent embryonic stem cells (mESCs).
Figure 2.8: RRBS analysis of genomic methylation levels in mouse induced totipotent stem cells (ciTotiSCs) and mouse pluripotent embryonic stem cells (mESCs).

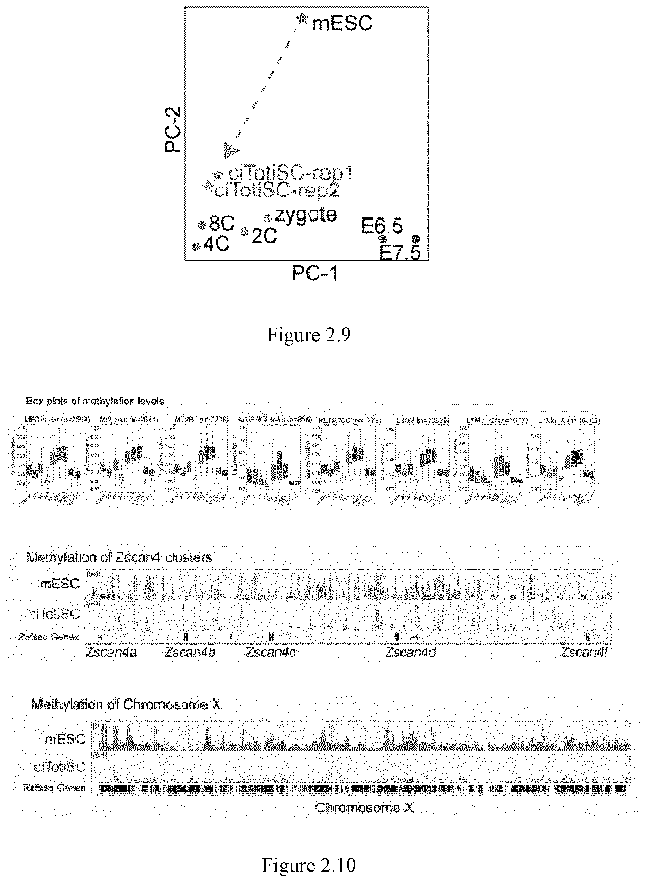

Figure 2.9: Global methylation principal component analysis of mouse induced totipotent stem cells (ciTotiSCs) and mouse pluripotent embryonic stem cells (mESCs) based on RRBS data.
Figure 2.10: Analysis of methylation levels near specific sites in the genomes of mouse induced totipotent stem cells (ciTotiSCs) and mouse pluripotent embryonic stem cells (mESCs).

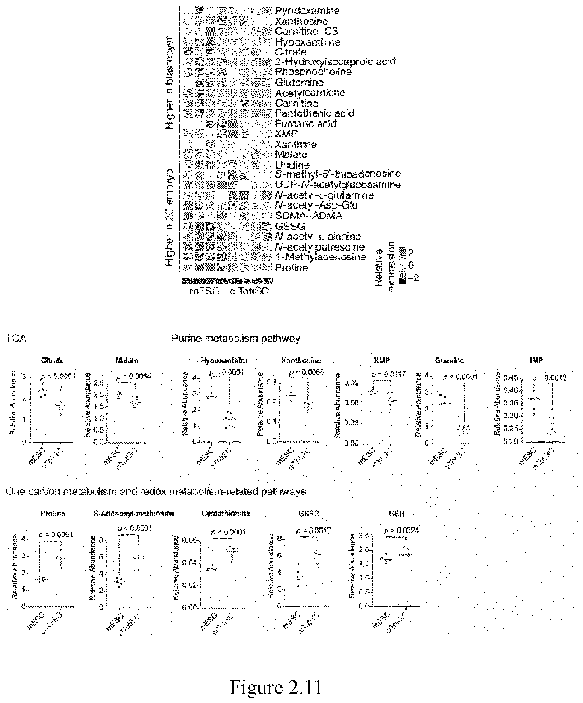

Figure 2.11: Analysis of the metabolome of mouse induced totipotent stem cells (ciTotiSCs).

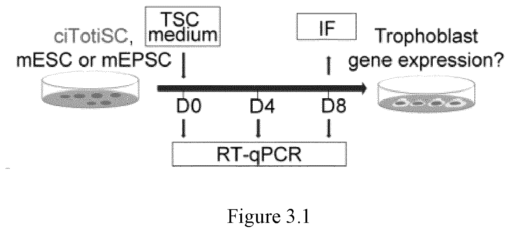

Figure 3.1: Schematic diagram of the experimental process of trophectoderm stem cell differentiation.

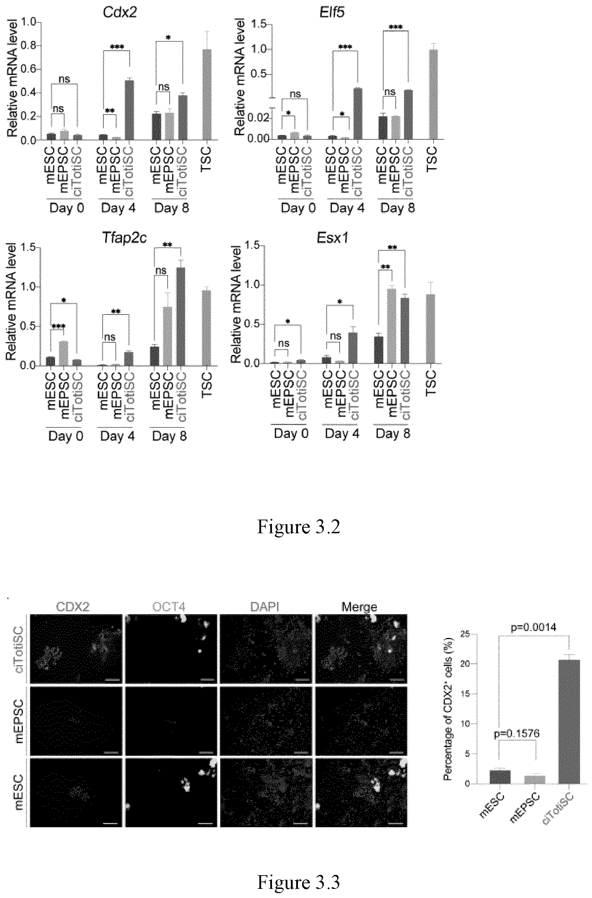

Figure 3.2: Detection of transcription of mouse trophectoderm stem cell-specific genes in mouse induced totipotent stem cells (ciTotiSCs), mouse embryonic stem cells (mESCs), and mouse expanded potential pluripotent stem cells (mEPSCs) by RT-qPCR.
Figure 3.3: Detection of mouse trophectoderm stem cell-specific protein expression in mouse induced totipotent stem cells (ciTotiSCs), mouse embryonic stem cells (mESCs) and mouse expanded potential pluripotent stem cells (mEPSCs) by immunofluorescence staining.

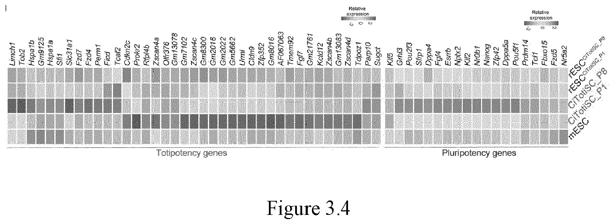

Figure 3.4: Analysis of the expression of totipotency and pluripotency genes in mouse induced totipotent stem cells (ciTotiSCs) of different passages (P1-P8) after being changed into mESC medium (2i/LIF).

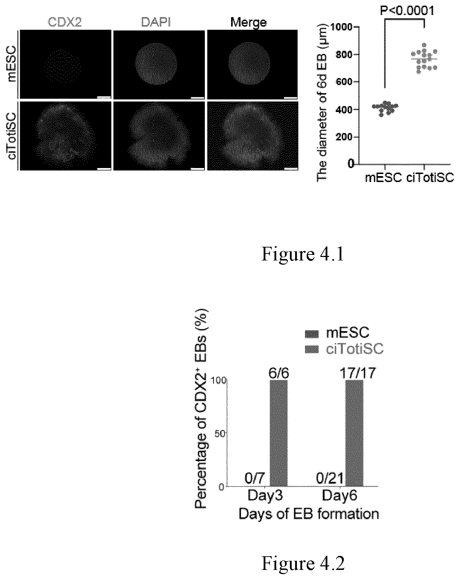

Figure 4.1: Immunofluorescence staining analysis of embryoid bodies (EBs) derived from mouse induced totipotent stem cells (ciTotiSCs) and mouse embryonic stem cells (mESCs).
Figure 4.2: Statistics of the proportion of all detected CDX2-positive cells in embryoid bodies.

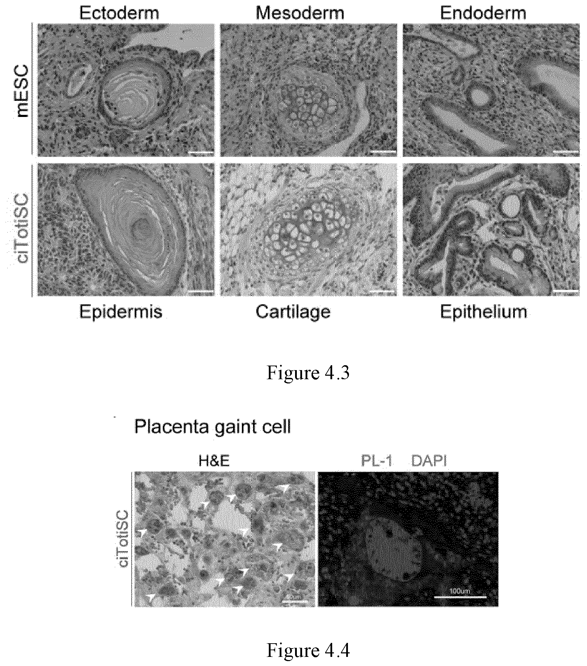

Figure 4.3: Analysis of the three-germ-layer-differentiation ability of teratomas derived from mouse induced totipotent stem cells (ciTotiSCs) and mouse embryonic stem cells (mESCs).
Figure 4.4: Analysis of teratoma extra-embryonic lineage differentiation ability of mouse induced totipotent stem cells (ciTotiSCs).

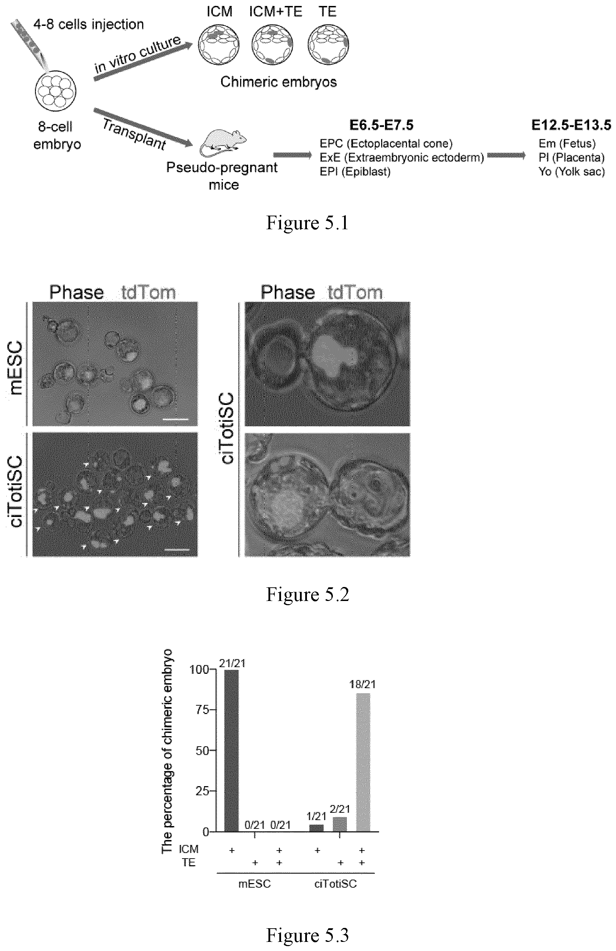

Figure 5.1: Schematic diagram of the chimeric assay process.
Figure 5.2: Chimeric ability of mouse totipotent stem cells (ciTotiSCs) and mouse embryonic stem cells (mESCs) at E4.5.
Figure 5.3: Statistics of chimerism ratio in mouse induced totipotent stem cells (ciTotiSCs) and mouse embryonic stem cells (mESCs) in the inner cell mass (ICM) and trophectoderm (TE).

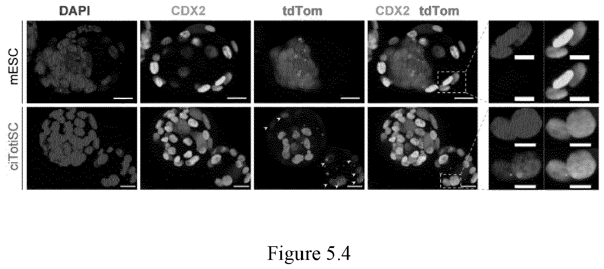

Figure 5.4: Confirmation of classic marker staining of chimeric embryonic trophectoderm (TE) in mouse induced totipotent stem cells (ciTotiSCs) and mouse embryonic stem cells (mESCs).

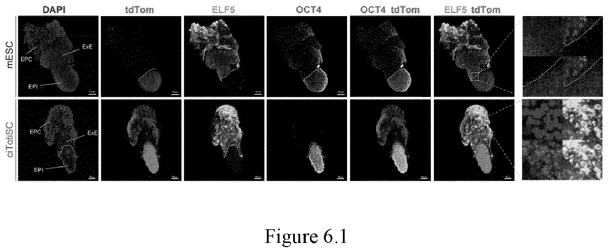

Figure 6.1: Immunofluorescence staining analysis in chimeric embryos of mouse induced totipotent stem cells (ciTotiSCs) and mouse embryonic stem cells (mESCs) developed to E7.5.

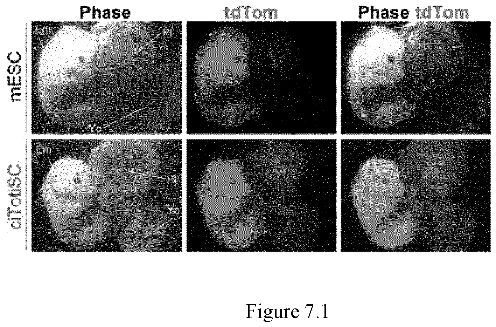

Figure 7.1: Chimeric status analysis in chimeric embryos of mouse induced totipotent stem cells (ciTotiSCs) and mouse embryonic stem cells (mESCs) developed to E12.5.

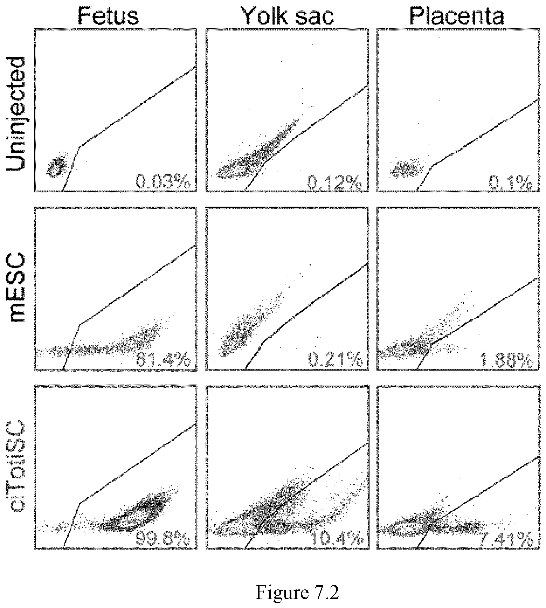

Figure 7.2: Flow cytometry analysis of the chimerism ratio of mouse induced totipotent stem cells (ciTotiSCs) and mouse embryonic stem cells (mESCs) in each tissue of E12.5.

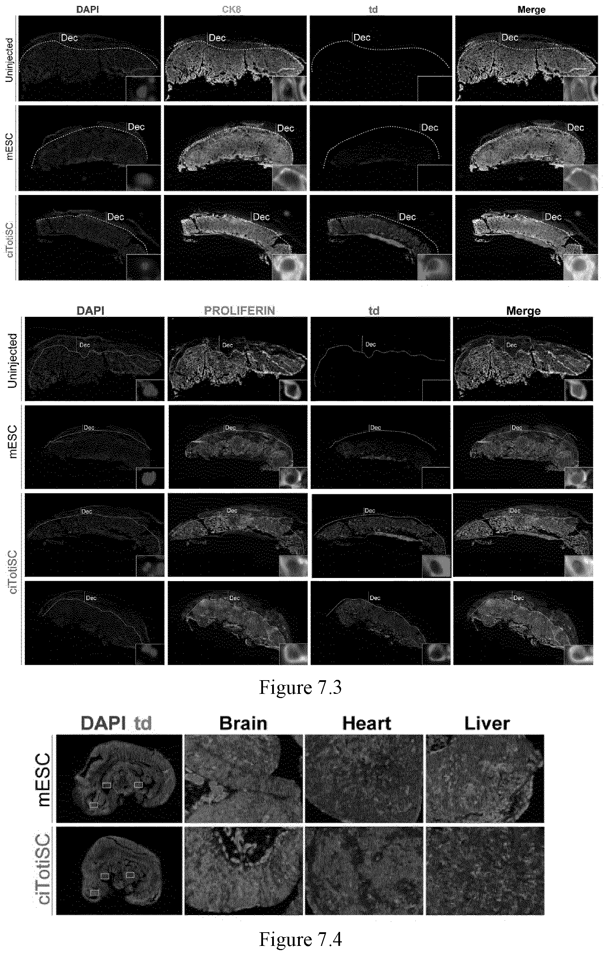

Figure 7.3: Immunofluorescence staining of E12.5 chimeric placental cryosections of mouse induced totipotent stem cells (ciTotiSCs) and mouse embryonic stem cells (mESCs) for analysis of co-localization of chimeric cells with the placental extra-embryonic lineage markers CK8 and proliferin.
Figure 7.4: Analysis of the chimeric ability of mouse induced totipotent stem cells (ciTotiSCs) and mouse embryonic stem cells (mESCs) in the three endodermal germ layers (mesoderm, endoderm, ectoderm) of E12.5.

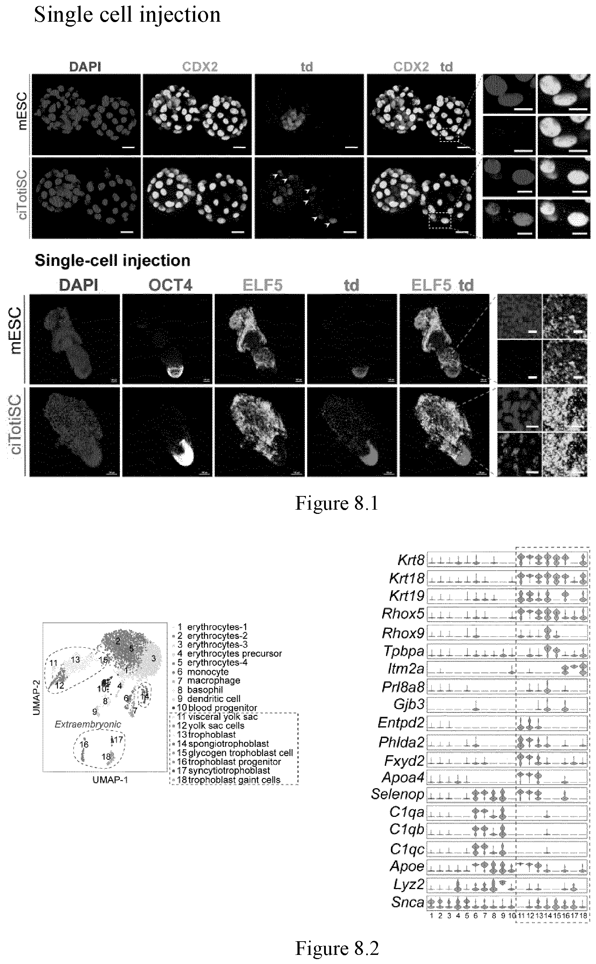

Figure 8.1: Detection of developmental potential of a single mouse induced totipotent stem cells (ciTotiSC).
Figure 8.2: Analysis of cell types formed by extra-embryonic and intra-embryonic chimerism of E12.5 in mouse induced totipotent stem cells (ciTotiSC).

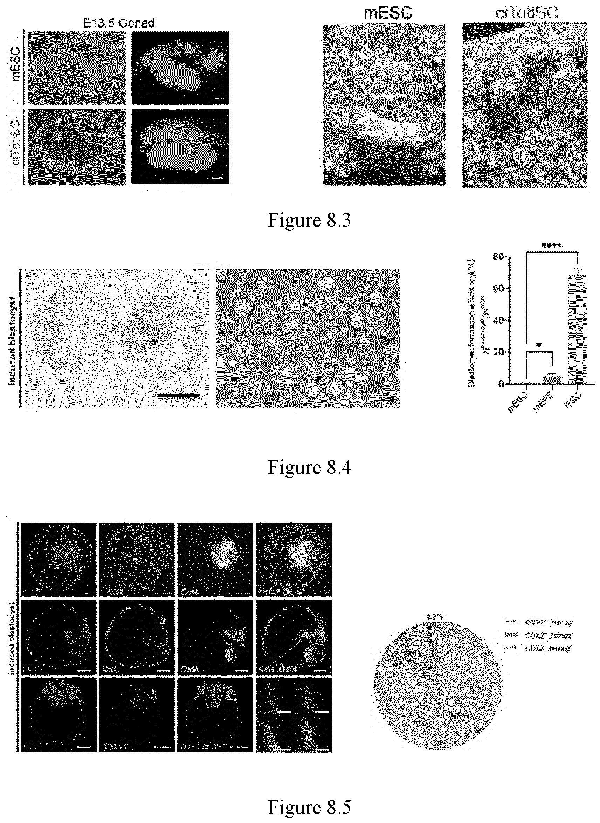

Figure 8.3: Mouse induced totipotent stem cells (ciTotiSCs) have the ability to chimerize into the reproductive ridge and produce healthy chimeric offspring.
Figure 8.4: Induced blastocysts obtained from mouse induced totipotent stem cells (ciTotiSCs).
Figure 8.5: Mouse blastocysts induced by mouse induced totipotent stem cells (ciTotiSCs) have three cell lineages of normal blastocysts in vivo.

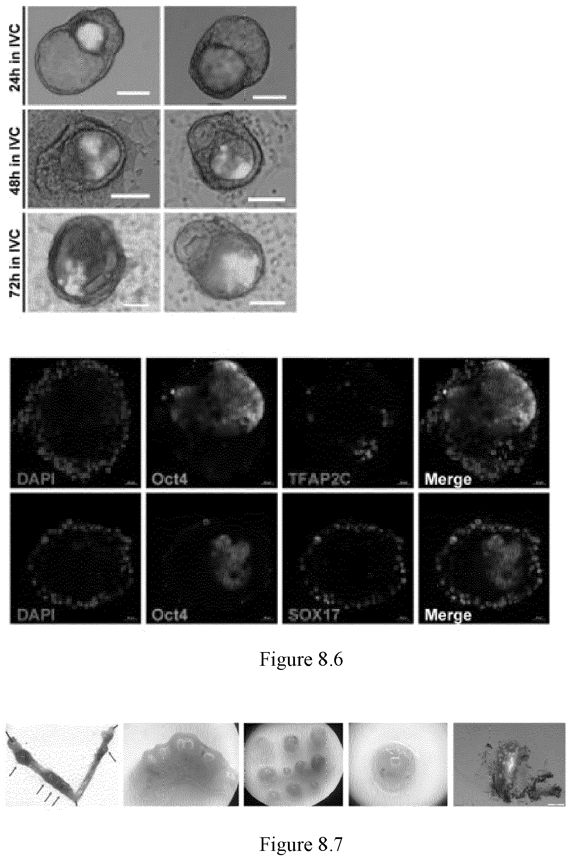

Figure 8.6: Mouse blastocysts induced by mouse induced totipotent stem cells (ciTotiSCs) can be developed after implantation in vitro.
Figure 8.7: Mouse blastocysts induced by mouse induced totipotent stem cells (ciTotiSCs) is implanted in the mouse uterus and further developed in vivo.

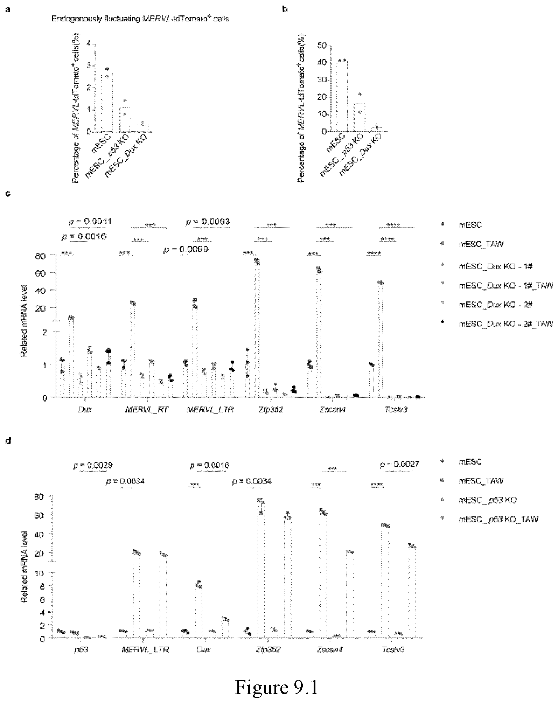

Figure 9.1: The role of Dux and p53 in the induction of totipotent stem cells (ciTotiSCs).

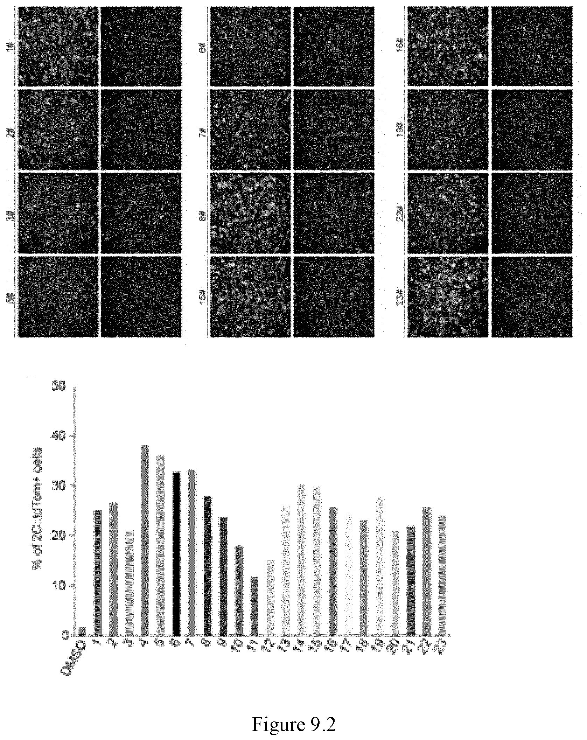

Figure 9.2: The 2C:tdTomato+ cell ratio of mouse totipotent stem cells (ciTotiSCs) induced by a combination of various small molecules.

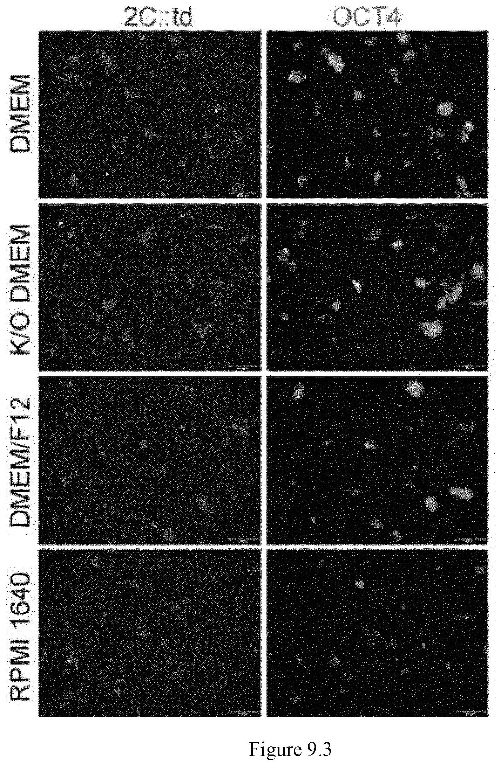

Figure 9.3: The 2C:tdTomato+ and OCT4 expression test for various commonly used basal media that are used to induce and generate mouse totipotent stem cells (ciTotiSCs).

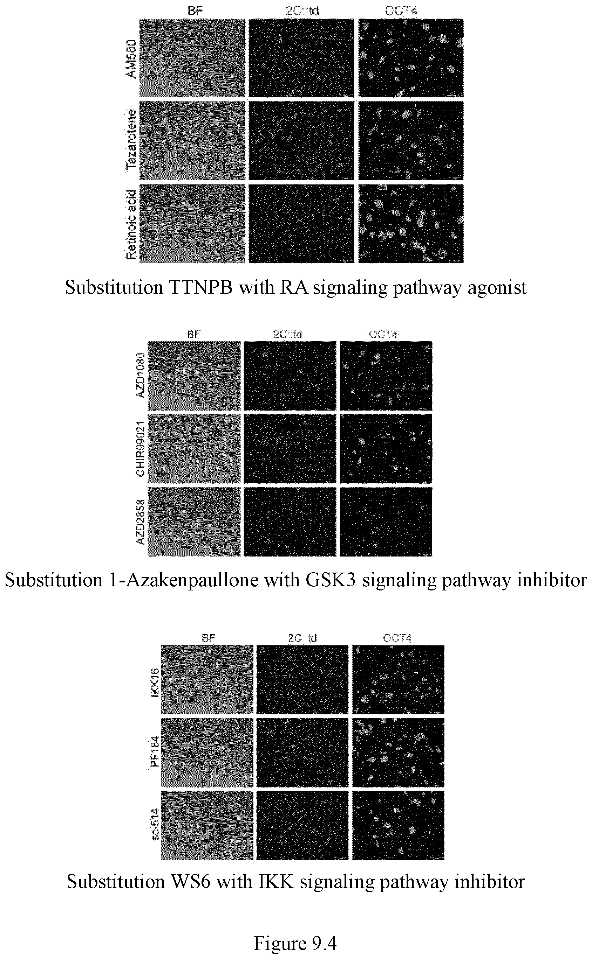

Figure 9.4: Individual replacement of some small molecule reprogramming reagents and testing of their performance in inducing mouse totipotent stem cells (ciTotiSCs).

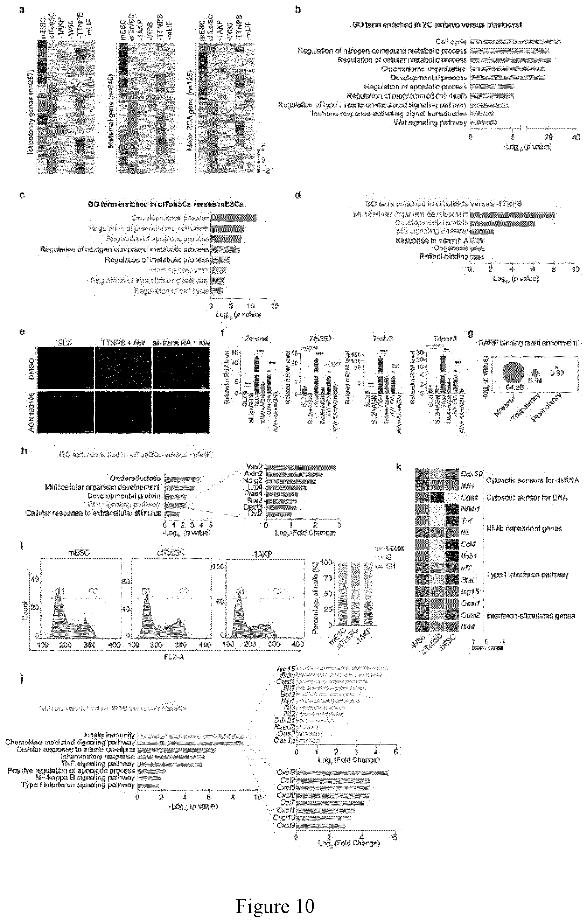

Figure 10: Effects of various small molecules on gene expression in totipotent cell induction.

### DETAILED DESCRIPTION OF THE INVENTION

### A. overview

Existing methods for the production and maintenance of totipotent stem cells have not yet achieved totipotent stem cells that meet one or more criteria in the recognized definition of totipotency, let alone the totipotent stem cells for industrial or clinical applications. The invention achieves the induction of pluripotent stem cells to produce the totipotent stem cells meeting one or more criteria in the recognized definition.

The totipotent stem cells induced and cultured by the inventors exhibit 1) transcriptome characteristics similar to those of mouse embryo zygotes and blastomeres of two-cell phase, transforming into the similar levels of totipotent cells *in vivo* in terms of chromatin accessibility, DNA methylation level and cell metabolism mode; 2) as demonstrated by the direct differentiation of monolayer cells *in vitro,* embryoid body differentiation in suspension and teratoma differentiation *in vivo* assays, the totipotent stem cells induced and cultured by the inventors have the ability to differentiate into extra-embryonic cells which is not possessed by pluripotent stem cells. An *in vivo* chimerism experiment also strongly prove that the totipotent stem cells induced and cultured by the inventors have high-efficiency bidirectional development potential to both intra-embryonic and extra-embryonic tissues; 3) more importantly, the induced totipotent stem cells can be independently induced and developed into mouse blastocysts *in vitro,* and correctly express mouse blastocyst marker genes. The induced blastocyst can show a series of characteristics after embryo implantation when continuously culturing *in vitro,* and the induced blastocyst can also be implanted into the uterus for continue development after being transplanted into mice. Therefore, the induced mouse totipotent stem cells have the potential to independently develop into an intact living body, but not through a traditional sperm-oocyte binding process.

Therefore, the compositions and methods envisaged herein make it possible to produce qualified totipotent stem cells that are suitable for industrial and clinical applications.

Unless otherwise specified, the implementation of the present invention will utilize conventional methods of chemistry, biochemistry, organic chemistry, molecular biology, microbiology, recombinant DNA technology, genetics, immunology, cell biology, stem cell protocol, cell culture and transgenic biology in the art, many of which are described below for illustrative purposes. Such techniques are fully explained in the literature. See, for example, sambrook, et al, molecular clocking: a laboratory manual (3rd edition, 2001); Sambrook, et al., Molecular Cloning: A Laboratory Manual (2nd Edition, 1989); Maniatis et al, Molecular Cloning: A Laboratory Manual (1982); Ausubel et al., Current Protocols in Molecular Biology (John Wiley and Sons, updated July 2008); Short Protocols in Molecular Biology: A Compendium of Methods from Current Protocols in Molecular Biology, Greene Pub. Associates and Wiley-Interscience; Glover, DNA Cloning: A Practical Approach, vol. I & II (IRL Press, Oxford, 1985); Anand, Techniques for the Analysis of Complex Genomes, (Academic Press, New York, 1992); Guthrie and Fink, Guide to Yeast Genetics and Molecular Biology (Academic Press, New York, 1991); Oligonucleotide Synthesis (N. Gait, Ed., 1984); Nucleic Acid Hybridization (B. Hames & S. Higgins, Eds., 1985); Transcription and Translation (B. Hames & S. Higgins, Eds., 1984); Animal Cell Culture (R. Freshney, Ed., 1986); Perbal, A Practical Guide to Molecular Cloning (1984); Fire et al., RNA Interference Technology: From Basic Science to Drug Development (Cambridge University Press, Cambridge, 2005); Schepers, RNA Interference in Practice (Wiley-VCH, 2005; Engelke, RNA Interference (RNAi): The Nuts & Bolts of siRNA Technology (DNA Press, 2003); Gott, RNAInterference, Editing, and Modification: Methods and Protocols (Methods in Molecular Biology; Human Press, Totowa, NJ, 2004); Sohail, Gene Silencing by RNA Interference: Technology and Application (CRC, 2004); Clarke and Sanseau, microRNA: Biology, Function & Expression (Nuts & Bolts series; DNA Press, 2006); Immobilized Cells And Enzymes (IRL Press, 1986); the treatise, Methods In Enzymology (Academic Press, Inc., N.Y); Gene Transfer Vectors For Mammalian Cells (J. H. Miller and M. P. Calos eds., 1987, Cold Spring Harbor Laboratory); Harlow and Lane, Antibodies, (Cold Spring Harbor Laboratory Press, Cold Spring Harbor, N.Y., 1998); Immunochemical Methods In Cell And Molecular Biology (Mayer and Walker, eds., Academic Press, London, 1987); Handbook Of Experimental Immunology, Volumes I-IV (D. M. Weir and C. Blackwell, eds., 1986); Riott, Essential Immunology, 6th Edition, (Blackwell Scientific Publications, Oxford, 1988); Embryonic Stem Cells: Methods and Protocols (Methods in Molecular Biology) (Kurstad Turksen, Ed., 2002); Embryonic Stem Cell Protocols: Volume I: Isolation and Characterization (Methods in Molecular Biology) (Kurstad Turksen, Ed., 2006); Embryonic Stem Cell Protocols: Volume II: Differentiation Models (Methods in Molecular Biology (Kurstad Turksen, Ed., 2006); Human Embryonic Stem Cell Protocols (Methods in Molecular Biology) (Kursad Turksen Ed., 2006); Mesenchymal Stem Cells: Methods and Protocols (Methods in Molecular Biology) (Darwin J. Prockop, Donald G. Phinney, and Bruce A. Bunnell Eds., 2008); Hematopoietic Stem Cell Protocols (Methods in Molecular Medicine) (Christopher A. Klug, and Craig T. Jordan Eds., 2001); Hematopoietic Stem Cell Protocols (Methods in Molecular Biology) (Kevin D. Bunting Ed., 2008) Neural Stem Cells: Methods and Protocols (Methods in Molecular Biology) (Leslie P. Weiner Ed., 2008); Hogan et al, Methods of Manipulating the Mouse Embryo (2nd Edition, 1994); Nagy et al, Methods of Manipulating the Mouse Embryo (3rd Edition, 2002), And The Zebrafish book. A guide for the laboratory use of zebrafish (Danio rerio), 4th Ed., (Univ. of Oregon Press, Eugene, OR, 2000).

All publications, patents and applications cited herein are incorporated herein by reference in their entirety.

### B. definitions

Unless otherwise defined, all technical and scientific terms used herein have the same meaning as commonly understood by one of ordinary skill in the art to which this invention belongs. For the purpose of the present invention, the following terms are defined below.

The articles "a", "an" and "the" used herein refer to one or more than one (i.e., at least one) grammatical objects of the articles. For example, "an element" means one element or more than one element.

The use of alternatives (e.g., "or") should be understood to mean either, both, or any combination thereof.

The terms "and/or" should be understood to mean either or both of the alternatives.

As used herein, the term "about" or "approximately" means a number, level, value, amount, frequency, percentage, scale, size, quantity, weight or length changed by up to 15%, 10%, 9%, 8%, 7%, 6%, 5%, 4%, 3%, 2% or 1% as compared to a reference number, level, value, amount, frequency, percentage, scale, size, quantity, weight or length. In one embodiment, the term "about" or "approximately" means a range of a number, level, value, quantity, frequency, percentage, scale, size, amount, weight or length within ±15%, ±10%, ±9%, ±8%, ±7%, ±6%, ±5%, ±4%, ±3%, ±2% or ±1% of a reference number, level, value, quantity, frequency, percentage, scale, size, amount, weight or length.

As used herein, the term "substantially" or "essentially" means a number, level, value, quantity, frequency, percentage, scale, size, amount, weight or length being about 90%, 91%, 92%, 93%, 94%, 95%, 96%, 97%, 98% or 99% or more as compared to a reference number, level, value, quantity, frequency, percentage, scale, size, amount, weight or length. In one embodiment, the term "substantially same" means a range of number, level, value, quantity, frequency, percentage, scale, size, amount, weight or length that is approximately the same as a reference number, level, value, quantity, frequency, percentage, scale, size, amount, weight or length range.

As used herein, the term "substantially free of', when used to describe a composition, such as a cell population or a culture medium, means a composition that does not contain a specified substance, such as 95%, 96%, 97%, 98%, 99% free of the specified substance, or undetectable as measured by a conventional means. Similar meanings can be applied to the term "absent", meaning the absence of a specific substance or component of the composition.

As used herein, the term "appreciable" means a range of number, level, value, amount, frequency, percentage, scale, size, quantity, weight or length that can be easily detected by one or more standard methods. The terms "not-acceptable" and "unappreciable" and equivalents means a range of number, level, value, amount, frequency, percentage, scale, size, quantity, weight or length that cannot be easily detected or undetectable by standard methods. In one embodiment, an event is not appreciable if it occurs at a frequency of less than 5%, 4%, 3%, 2%, 1%, 0.1%, 0.01%, 0.001% or less.

Throughout this specification, unless otherwise required by the context, the terms "comprising", "including", "containing" and "having" shall be understood to imply the inclusion of the stated steps or elements or a group of the steps or elements, but not exclusion of any other steps or elements or a group of steps or elements. In certain embodiments, the terms "comprising", "including", "containing" and "having" are used synonymously.

The phrase "consisting of" means including but limited to any elements following the phrase "consisting of". Therefore, the phrase "consisting of" indicates that the elements listed are required or mandatory, and no other elements can exist.

The phrase "consisting essentially of" means including any elements listed following the phrase "consisting essentially of", and is limited to other elements that do not interfere with or contribute to the activities or actions specified in the disclosure of the listed elements. Therefore, the phrase "consisting essentially of" indicates that the elements listed are required or mandatory, but no other elements are optional and may or may not exist depending on whether they affect the activities or actions of the listed elements.

Throughout this specification, reference to "one embodiment", "an embodiment", "a particular embodiment", "a related embodiment", "a certain embodiment", "an additional embodiment" or "a further embodiment" or a combination thereof means that a particular feature, structure, or characteristic described in connection with the embodiment is included in at least one embodiment of the present invention. Therefore, the occurrence of the aforementioned phrases in various parts of the whole specification does not necessarily refer to the same embodiment. Furthermore, the specific features, structures, or characteristics may be combined in any suitable manner in one or more embodiments.

The term *"ex vivo"* generally means activities occurring outside the body of an organism, such as experiments or measurements performed in or on a living tissue in an artificial environment outside the body of the organism, preferably with a minimal change in natural conditions. In certain embodiments, an *"ex vivo"* procedure involves living cells or tissues obtained from the organism, cultured in a laboratory instrument, usually under sterile conditions and generally for several hours or up to about 24 hours, but including up to 48 or 72 hours, depending on the environment. In some embodiments, such tissue or cells may be collected and frozen, and subsequently thawed for *ex vivo* processing. The tissue culture experiments or procedures using living cells or tissues for more than several days are generally deemed as *"in vitro",* although in certain embodiments, the term may be used interchangeably with "ex *vivo".*

The term *"in vivo"* generally means activities occurring in a living organism.

As used herein, the terms "reprogramming" or "dedifferentiation" or "increasing cell potential" or "increasing developmental potential" refer to a method or process that increase cell potential or dedifferentiate cells into a less differentiated state. For example, the cells with increased cell potential have more developmental plasticity (i.e., can differentiate into more cell types) than the same cells in a non-reprogrammed state. In other words, reprogrammed cells are those in a less differentiated state than the same cells in a non-reprogrammed state. In some embodiments, the reprogramming includes reprogramming pluripotent stem cells into totipotent stem cells. In some embodiments, the reprogramming includes reprogramming non-pluripotent stem cells into pluripotent stem cells. In some embodiments, the reprogramming includes reprogramming non-pluripotent stem cells into pluripotent stem cells.

As used herein, the term "potential" means the sum of all developmental options available to a cell (i.e., developmental potential). One of ordinary skill in the art will recognize that cell potential is a continuum ranging from the cells with most plasticity (i.e., totipotent stem cells, which have the most developmental potential) to the cells with least plasticity (i.e., terminally differentiated cells, which have the least developmental potential). Continuum of cell potential includes, but is not limited to, totipotent cells, pluripotent or multipotent cells, oligopotent cells, monopotent cells, and terminally differentiated cells.

As used herein, the term "pluripotent" means the ability of cells to generate all lineages of an organism or a body (i.e., embryoid). For example, embryonic stem cells are a class of pluripotent stem cells capable of forming cells from each of the three germ layers (ectoderm, mesoderm, and endoderm).

As used herein, the term "totipotent" means cells that conform to one or more, preferably two, and more preferably all three of the following: 1) the cells are similar to totipotent embryonic cells, i.e., zygotes and two-cell blastomeres, in terms of transcription level; 2) further, the cells have the potential of bidirectional development to both intra-embryonic and extra-embryonic cell types; 3) more further and most strictly, one cell can develop into a complete embryo or a living individual. For example, as proved by the present invention, the induced totipotent stem cells of the present invention exhibit 1) the transcriptome characteristics similar to those of mouse embryo zygotes and 2-cell blastomeres, and are transformed into the levels similar to those of totipotent cells *in vivo* in terms of chromatin accessibility, DNA methylation level and cell metabolism mode; 2) as demonstrated by the direct differentiation of monolayer cells *in vitro,* embryoid body differentiation in suspension and teratoma differentiation *in vivo* assays, the totipotent stem cells induced and cultured by the inventors have the ability to differentiate into extra-embryonic cells which is not possessed by pluripotent stem cells. An *in vivo* chimerism experiment also strongly prove that the totipotent stem cells induced and cultured by the inventor have high-efficiency bidirectional development potential to both intra-embryonic and extra-embryonic tissues; 3) more importantly, the induced totipotent stem cells can be independently induced and developed into mouse blastocysts *in vitro,* and correctly express mouse blastocyst marker genes. The induced blastocyst can show a series of characteristics after embryo implantation when continuously culturing *in vitro,* and the induced blastocyst can also be implanted into the uterus for continue development after being transplanted into mice. Therefore, the induced mouse totipotent stem cells have the potential to independently develop into an intact living body, but not through a traditional sperm-oocyte binding process.

Totipotency can be determined, in part, by evaluating the totipotency characteristics of cells. The totipotency characteristics include, but are not limited to: (1) the morphology of totipotent stem cells; (2) increased transcription of totipotent transcription markers, such as MERVL, Zscan4c, Zscan4d, Zscan4f, Zfp352, Tcstv1, Tcstv3, Teme92, Gm6763; (3) reduced transcription of pluripotent transcription markers such as POU5f1, ZFP42, NANOG, KLF4, ESRRB; (4) the ability to differentiate into embryonic cell types; (5) the ability to differentiate into extra-embryonic cell types; and (6) the ability to develop into an independent individual. The induced totipotent stem cells of the present invention can be characterized by one or more of these characteristics. These totipotent characteristics of the induced totipotent stem cells of the present invention can be compared with natural or artificial totipotent stem cells and/or natural or artificial pluripotent stem cells, for example, comparing with natural totipotent stem cells of zygotes and/or 2-cell blastomeres, and/or with embryonic stem cells or induced pluripotent stem cells; or comparing with control totipotent and/or pluripotent stem cells without the same culture conditions.

In certain embodiments, the increased or decreased transcription of transcription markers caused by a given culture condition may be an increase or decrease by at least 1, 2, 3, 4, 5, 6, 7, 8, 9, 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900, 950, 1000% or more compared to an appropriate control.

As used herein, "gene expression" or "gene transcription" means the relative levels of gene expression/transcription and/or expression/transcription patterns in a biological sample such as totipotent cells or a cell population comprising totipotent cells. In certain embodiments, totipotent cells are induced totipotent stem cells.

The present disclosure covers any method available in the art for detecting the expression/transcription of genes characterizing the cells of the present invention. As used herein, the term "detecting expression/transcription" means determining the amount or presence of RNA transcripts of genes or expression products thereof. Methods for detecting gene expression/transcription, i.e., gene expression/transcription profile assays, include polynucleotide-based hybridization analysis, polynucleotide-based sequencing, immunohistochemistry, and proteomics-based methods. These methods generally detect an expression/transcription product (e.g., mRNA) of a gene of interest. In some embodiments, a PCR-based method such as reverse transcription PCR (RT-PCR) (Weis et al., TIG8:263-64, 1992) and an array-based method such as a microarray (Schena et al., Science 270:467-70, 1995) are used.

"Adherence" refers to the attachment of cells to a container in the presence of an appropriate culture medium, e.g., cell attachment to a sterile plastic (or coated plastic) cell culture dish or flask. Certain types of cell cannot maintain or grow in culture, unless they adhere to a cell culture container. Certain types of cell ("non-adherent cells") maintain and/or proliferate in culture without adherence to the wall.

"Culture" or "cell culture" refers to the maintenance, growth and/or differentiation of cells in an *in vitro* environment. "Cell culture medium", "culture medium", "culture media", "medium", "supplement" and "culture medium supplement" refer to nutritional compositions for culturing cell cultures.

"Culture" or "cell culture" refers to a material to be cultured such as cells, and/or a culture medium with the material to be cultured such as cells therein.

"Cultivate" refers to the maintenance, reproduction (growth) and/or differentiation of cells outside a tissue or body, for example, in a sterile plastic (or coated plastic) cell culture dish or flask. "Cultivation" can use a culture medium as a source of nutrients, hormones and/or other factors that facilitate cell reproduction and/or maintenance.

As used herein, "dissociated" cells refer to cells that have been substantially separated or purified from other cells or surfaces (e.g., the surface of a culture plate). For example, cells can be dissociated from an animal or a tissue by a mechanical or enzymatic method. Alternatively, cells aggregated *in vitro* may be enzymatically or mechanically dissociated from each other, for example by dissociation into clusters, single cells, or a suspension of a mixture of single cells and clusters. In another alternative embodiment, adherent cells are dissociated from the culture plate or other surface. Dissociation may therefore involve disrupting the interaction of cells with extracellular matrix (ECM) and a substrate (e.g., a culture surface), or disrupting ECM between cells.

As used herein, the term "enrich" means increasing the amount of a specified component in a composition such as a cell composition, and when used to describe a composition such as a cell population, "enriched" means a cell population having a proportionally increased amount of the specified component as compared to the proportion of such a component in the cell population prior to enrichment. For example, a composition such as a cell population can be enriched for a target cell type (i.e., cells having specified characteristics) and thus has an increased proportion or percentage of the target cell type compared to the proportion of target cells present in the cell population prior to enrichment. A cell population can be enriched for a target cell type by a cell selection and sorting method known in the art. In some embodiments, the cell population is enriched by a sorting or selection method. In a particular embodiment, the method of enrichment for the target cell population will enrich the cell population by at least about 20% with respect to the target cell population, meaning that the enriched cell population proportionally comprises about 20% more target cell types than the cell population before the cell population is enriched. In one embodiment, the method of enrichment for the target cell population causes the cell population to be proportionally enriched for the target cell population by at least about 30+%, 40+%, 50+%, 60+%, 70+%, 80%, 85%, 90%, 95%, 97%, 98% or 99%, or at least about 98%, or in particular embodiments, about 99%.

In certain embodiments, the cell population is enriched in terms of the amount of totipotent cells or cells exhibiting totipotency characteristics. In particular embodiments of the present invention, a cell population undergoing reprogramming is enriched for the target cells having totipotency characteristics, such as the expression of totipotency markers including, but not limited to MERVL, Zscan4c, Zscan4d, Zscan4f, Zfp352, Tcstv1, Tcstv3, Teme92, Gm6763.

In certain embodiments, enriched cells comprise different gene or protein expression profiles, such as cell surface expression of one or more totipotent markers such as MERVL, Zscan4c, Zscan4d, Zscan4f, Zfp352, Tcstv1, Tcstv3, Teme92, Gm6763. In some embodiments, in one embodiment, the cell population comprises at least about 5%, 10%, 15%, 20%, 25%, 30%, 40%, 50%, 70%, 75%, 80%, 90%, 95%, 97%, 98%, or 99% of the enriched cells such as totipotent cells.

Therefore, in some embodiments, the method of enriching totipotent cells from the cell population includes sorting the cell population based on the cell surface expression of totipotent markers such as MERVL, Zscan4c, Zscan4d, Zscan4f, Zfp352, Tcstv1, Tcstv3, Teme92, Gm6763, and collecting cell fractions expressing such markers to obtain a cell population rich in totipotent cells. In other embodiments, the cell population is sorted based on the cell surface expression of pluripotent cell markers such as POU5f1, ZFP42, NANOG, KLF4, ESRRB, and the cell population of such cells is depleted to obtain a cell population rich in totipotent cells, so that the cell population is rich in totipotent cells.

As used herein, "feeder cells" or "feeders" is used to describe a type of cell co-cultured with a second type of cell to provide an environment in which the second type of cell can grow, because the feeder cells provide growth factors and nutrients to support the second type of cell. Feeder cells are optionally from a different species than the cells they support. For example, certain types of human cells including stem cells can be supported by primary cultures of mouse embryonic fibroblasts and immortalized mouse embryonic fibroblasts. When co-culturing with other cells, feeder cells can generally be inactivated by irradiation or treatment with antimitotic agents such as mitomycin C to prevent their growth from exceeding the cells they support. Without being limited to the foregoing, a specific feeder cell type may be human feeder cells, such as human skin fibroblasts. Another feeder cell type may be mouse embryonic fibroblast (MEF).

As used herein, a "feeder-free" (FF) environment refers to an environment which is substantially free of feeder cells and/or which is not preconditioned by feeder cell culturing, such as a cell culture or a culture medium. The "preconditioned" medium refers to a medium in which feeder cells have been harvested after culturing in the medium for a period of time, for example at least one day. The preconditioned medium comprises many medium substances, including growth factors and cytokines secreted by the feeder cells cultured in the medium.

Genome stability refers to the ability of cells to faithfully replicate DNA and maintain the integrity of DNA replication process. As used herein, the terms "genome-stable cells" and "cells having genome stability" refer to cells that exhibit certain frequencies of mutations and chromosomal abnormalities (e.g., translocation, aneuploidy, copy number variation and duplication), which are substantially similar to the frequencies of mutations and chromosomal abnormalities relative to normal human cells.

"Component" refers to any compound or other materials that can be used in a cell culture medium to maintain and/or promote cell growth and/or differentiation, regardless of its source being chemical or biological. The terms "component", "nutrient" and "ingredient" are used interchangeably. Conventional components for cell culture media may include, but are not limited to, amino acids, salts, metals, sugars, lipids, nucleic acids, hormones, vitamins, fatty acids, proteins, and the like. Other components for promoting and/or maintaining cell culture *ex vivo* or *in vitro* can be selected by those of ordinary skill in the art according to the requirement for desired effects.

"Isolation" refers to the separation and collection of a composition or material from its natural environment, such as the separation of individual cells or cell cultures from tissues or organisms. In one aspect, the cell population or composition is substantially free of cells and materials with which the cell population or composition is associated in nature. Regarding the target cells comprised in the total cell population, "isolated" or "purified" or "substantially pure" means the cell population is at least about 50%, at least about 75%, at least about 85%, at least about 90%, and in certain embodiments, at least about 95% pure. The purity of the cell population or composition can be evaluated by a suitable method well known in the art. For example, a substantially pure totipotent cell population means, with respect to totipotent cells comprised in the total cell population, the cell population is at least about 50%, at least about 75%, at least about 85%, at least about 90%, and in certain embodiments, at least about 95%, and in certain embodiments, about 98% pure.

"Passaging" refers to an operation of subdividing and spreading cells into multiple cell culture surfaces or vessels when the cells have proliferated to a desired level. In some embodiments, "passaging" refers to subdividing, diluting, and plating cells. When cells are passaged from the primary culture surface or vessel to a surface or container of the subsequent groups, the subsequent culture may be referred to herein as "subculture" or "first passage", etc. the operation of each subdivision and plating into a new culture vessel is considered as a passage.

"Plating" means placing one or more cells in a culture vessel such that the cells adhere to the cell culture vessel and spread over the cell culture vessel.

"Pluripotent factor" refers to a reagent that can increase the development potential of cells to the extent of pluripotency, either alone or in combination with other reagents. The pluripotent factors include, but are not limited to, polynucleotides, polypeptides and small molecules that can increase the development potential of cells to the extent of pluripotency. Exemplary pluripotent factors include, for example, transcription factors and small molecule reprogramming agents.

"Totipotent factor" refers to a reagent that can increase the development potential of cells to the extent of totipotency, either alone or in combination with other reagents. The totipotency factors include, but are not limited to polynucleotides, polypeptides and small molecules that can increase the development potential of cells to the extent of totipotency. Exemplary totipotent factors include, for example, transcription factors and small molecule reprogramming agents.

"Proliferation" refers to the property that a cell divides into two substantially equivalent cells or the number of cell populations increases (e.g., for replication).

"Reproduction" refers to the growth (e.g., reproduction via cell proliferation) of cells outside the tissue or body, for example, in a sterile container such as a plastic (or coated plastic) cell culture dish or flask.

"Primary culture" refers to cells, tissues and/or cultures in which isolated cells are placed in a first culture vessel having a culture medium. However, the cells, tissues and/or cultures can be maintained and/or proliferated as long as the cells, tissues and/or cultures remain in the first container. The cells, tissues and/or cultures are referred to as primary cultures.

The terms "small molecule reprogramming agents" or "small molecule reprogramming compounds" are used interchangeably herein, and refer to small molecules that can increase the development potential of cells, either alone or in combination with other factors. The small molecules include, but are not limited to, nucleic acids, peptidomimetics, peptoids, carbohydrates, lipids, or other organic or inorganic molecules. Libraries of chemical and/or biological mixtures such as fungal, bacterial or algal extracts are known in the art, and in certain embodiments, can be used as small molecule sources.

### C. cells

In a particular embodiment, one or more cells can be cultured, dissociated, and passaged using the compositions and methods contemplated herein. In one embodiment, single cells are cultured, dissociated and passaged using the compositions and methods contemplated herein. In another embodiment, a cell population or multiple cells are cultured, dissociated and passaged using the compositions and methods contemplated herein.

Starting cells suitable for use in a particular embodiment may be derived from essentially any suitable source and may be heterogeneous or homogeneous with respect to cell type or totipotency state. Such suitable cells include fetal cells and adult cells. Additionally, such suitable cells may be mammalian in origin, e.g., from a rodent, feline, canine, porcine, goat, ovine, equine, bovine, or primate, e.g., human. In one embodiment, the cells are human cells.

The cells may be somatic cells, non-pluripotent, incomplete or partially pluripotent stem cells, pluripotent cells, oligopotent cells, unipotent cells, terminally differentiated cells, or a mixed population of cells comprising any combination of the foregoing. The pluripotent cells suitable for use in a particular embodiment include, but are not limited to, naturally occurring stem cells, embryonic stem cells, or iPSCs. A "mixed" population of cells is a population of cells with varying degrees of developmental potential. For example, a mixed population of cells may comprise cells that have undergone reprogramming such that the mixed population comprises pluripotent cells, partially pluripotent cells, and non-pluripotent cells, e.g., fully differentiated cells, such as somatic cells. In some embodiments, the pluripotent stem cells are used to induce the totipotent stem cells described herein. In some embodiments, the pluripotent stem cells are embryonic stem cells or induced pluripotent stem cells. In some embodiments, the pluripotent stem cells are reprogrammed from non-pluripotent stem cells. In some embodiments, the non-pluripotent stem cells are selected from the group consisting of somatic cells and/or adult stem cells. In some embodiments, reprogramming a non-pluripotent cell to a pluripotent stem cell comprises expressing within the non-pluripotent cell one or more reprogramming factors selected from the group consisting of Oct4, Sox2, Klf4 and c-Myc.

In one embodiment, the starting cell population is selected from the group consisting of adult or neonatal stem/progenitor cells. In a specific embodiment, the starting stem/progenitor cell population is selected from the group consisting of: mesodermal stem/progenitor cells, endodermal stem/progenitor cells, and ectodermal stem/progenitor cells.

Illustrative examples of mesodermal stem/progenitor cells include, but are not limited to: mesodermal stem/progenitor cells, endothelial stem/progenitor cells, bone marrow stem/progenitor cells, umbilical cord stem/progenitor cells, adipose tissue-derived stem/progenitor cells, hematopoietic cells Stem/progenitor cells (HSC), mesenchymal stem/progenitor cells, muscle stem/progenitor cells, kidney stem/progenitor cells, osteoblast stem/progenitor cells, chondrocyte stem/progenitor cells, and the like.

Illustrative examples of ectodermal stem/progenitor cells include, but are not limited to, neural stem/progenitor cells, retinal stem/progenitor cells, skin stem/progenitor cells, and the like.

Illustrative examples of endodermal stem/progenitor cells include, but are not limited to, liver stem/progenitor cells, pancreatic stem/progenitor cells, epithelial stem/progenitor cells, and the like.

In certain embodiments, the starting cell population may be a heterogeneous or homogeneous population of cells selected from the group consisting of islet cells, CNS cells, PNS cells, cardiomyocytes, skeletal muscle cells, smooth muscle cells, hematopoietic cells, osteocytes, liver cells, fat cells, kidney cells, lung cells, chondrocytes, skin cells, follicular cells, vascular cells, epithelial cells, immune cells, endothelial cells, and the like.

### D. A culture platform that can be used to induce totipotency and induce totipotent stem cells

Cell banking, disease modeling, and cell therapy applications have placed increasing demands on the production of high-quality totipotent cells. The present invention provides a culture platform that can be used to induce totipotency and to induce totipotent stem cells using specific small molecule reprogramming agents.

In one aspect the present invention provides a composition comprising:
(a) a RA signaling pathway activator; and
(b) one or more of a GSK-3 inhibitor, an IKK signaling pathway inhibitor, an HDAC inhibitor, a histone methyltransferase inhibitor, a Src kinase inhibitor, a cAMP activator, and a cellular metabolism modulator.

In another aspect, the present invention provides a kit comprising:
(a) a RA signaling pathway activator; and
(b) one or more of a GSK-3 inhibitor, an IKK signaling pathway inhibitor, an HDAC inhibitor, a histone methyltransferase inhibitor, a Src kinase inhibitor, a cAMP activator, and a cellular metabolism modulator.

In yet another aspect, the present invention provides (a) a RA signaling pathway activator; and (b) one or more of a GSK-3 inhibitor, an IKK signaling pathway inhibitor, an HDAC inhibitor, a histone methyltransferase inhibitor, a Src kinase inhibitor, a cAMP activator, and a cell metabolism modulator, for use in the production of induced pluripotent stem cells.

### 1. RA signaling pathway activator

The RA (retinoic acid) signaling pathway activator may be various agents capable of activating the RA pathway. Exemplary RA signaling pathway activators include, but are not limited to, TTNPB, Tretionin/RA/ATRA, AM580, Taza, 9-cis-RA, Acitretin, CD437, Tamibarotene, Tazarotene, retinoic acid, Isotretinoin, Acitretin sodium, ch55, and AC55649. In some embodiments, the RA signaling pathway activator is selected from the group consisting of TTNPB, Tretionin/RA/ATRA, AM580, Taza, 9-cis-RA, Acitretin, CD437, Tamibarotene, Tazarotene, retinoic acid, Isotretinoin, Acitretin sodium, ch55 and AC55649. Preferably, in some embodiments, the RA signaling pathway activator is TTNPB, as shown in the formula below:

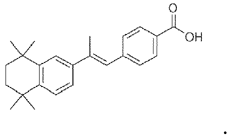

### 2. GSK-3 inhibitor

The GSK-3 (glycogen synthase kinase-3) inhibitor may be various agents capable of inhibiting GSK-3. The GSK-3 inhibitor includes, but is not limited to, 1-Azakenpaullone, AZD2858, CHIR99021 and AZD1080. In some embodiments, the GSK-3 inhibitor is selected from the group consisting of 1-Azakenpaullone, AZD2858, CHIR99021 and AZD1080. Preferably, in some embodiments, the GSK-3 inhibitor is 1-Azakenpaullone, as shown in the formula below:

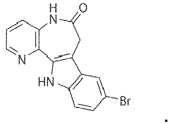

### 3. IKK signaling pathway inhibitor

The IKK (IKB kinase/NF-κB, IKK/NF-κB) signaling pathway inhibitor may be a variety of agents capable of inhibiting the IKK signaling pathway. Exemplary IKK signaling pathway inhibitors include, but are not limited to, WS6, sc-514, PF184, and IKK16. In some embodiments, the IKK signaling pathway inhibitor is selected from the group consisting of WS6, sc-514, PF184, and IKK16. Preferably, in some embodiments, the IKK signaling pathway inhibitor is WS6, as shown in the formula below:

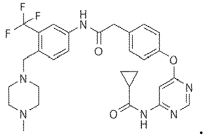

### 4. HDAC inhibitor

The HDAC (Histone deacetylase) inhibitor may be a variety of agents capable of inhibiting HDAC. Exemplary HDAC inhibitors include, but are not limited to, Trichostatin A (TSA), Valproic acid (VPA), Vorinostat (SAHA), and Entinostat (MS-275). In some embodiments, the HDAC inhibitor is selected from the group consisting of Trichostatin A (TSA), Valproic acid (VPA), Vorinostat (SAHA), and Entinostat (MS-275).

### 5. Histone methyltransferase inhibitor

The histone methyltransferase inhibitor may be a wide variety of agents capable of inhibiting histone methyltransferases. Exemplary histone methyltransferase inhibitors include, but are not limited to, BIX 01294, 3-deazaneplanocin A (DZNeP) HCl, A-366, UNC0638, and SGC 0946. In some embodiments, the histone methyltransferase inhibitor is selected from the group consisting of BIX 01294, 3-deazaneplanocin A (DZNeP) HCl, A-366, UNC0638, and SGC 0946.

### 6. Src kinase inhibitor

The Src kinase inhibitor may be a variety of agents capable of inhibiting Src. Exemplary Src kinase inhibitors include, but are not limited to, Dasatinib (BMS-354825), WH-4-023, Ponatinib (AP24534), Bosutinib (SKI-606). In some embodiments, the Src kinase inhibitor is selected from the group consisting of Dasatinib (BMS-354825), WH-4-023, Ponatinib (AP24534), Bosutinib (SKI-606).

### 7. cAMP activator

The cAMP activator may be a wide variety of agents capable of activating cAMP. Exemplary cAMP activators include, but are not limited to, olforsin (Forskolin, HL 362) and 8-Br-cAMP. In some embodiments, the cAMP activator is selected from the group consisting of Colforsin (Forskolin, HL 362) and 8-Br-cAMP.

### 8. Cell metabolism modulator

The cell metabolism modulator may be a wide variety of agents capable of modulating cellular metabolism. Exemplary cell metabolism modulators include, but are not limited to, 2-Deoxy-D-glucose (2-DG), sodium acetate, sodium L-lactate, and D-ribose. In some embodiments, the cell metabolism modulator is selected from the group consisting of 2-Deoxy-D-glucose (2-DG), sodium acetate, sodium L-lactate, and D-ribose.

### 9. The amounts of the components

The amount of the small molecule reprogramming agent in the composition, kit, medium or culture of the present invention may vary and may be optimized based on the specific culture conditions, including the specific molecules and combinations used, the type of cells cultured in the media, and the specific applications. In one embodiment, the small molecule reprogramming agent is present in the composition, kit, culture of the present invention at a concentration sufficient to induce totipotency, improve reprogramming efficiency, increase or maintain the potency of the cell, or induce or maintain basal state totipotency.

In some embodiments, the RA signaling pathway activator is present in the composition, kit, medium or culture of the present invention in an amount or concentration sufficient to induce totipotency, alone or in combination with other small molecule reprogramming agents. In some embodiments, the RA signaling pathway activator is present in the composition, kit, medium or culture of the present invention at a concentration of 0.01, 0.05, 0.1, 0.2, 0.3, 0.4, 0.5, 0.6, 0.7, 0.8, 0.9, 1.0, 1.1, 1.2, 1.4, 1.6, 1.8, 2.0, 2.2, 2.4, 2.6, 2.8, 3.0, 3.2, 3.4, 3.6, 3.8, 4.0, 4.2, 4.4, 4.6, 4.8, 5.0, 5.2, 5.4, 5.6, 5.8, 6.0, 6.2, 6.4, 6.6, 6.8, 7.0, 7.2, 7.4, 7.6, 7.8, 8.0, 8.2, 8.4, 8.6, 8.8, 9.0, 9.2, 9.4, 9.6, 9.8, 10.0 µM or higher or a range consisting of any two of the foregoing values. Preferably, the RA signaling pathway activator is present in the composition, kit, medium or culture of the present invention at a concentration of 0.05-5 µM, preferably 0.1-1 µM, more preferably 0.2 µM. Most preferably, in one embodiment, the TTNPB is present in the composition, kit, medium or culture of the present invention at a concentration of 0.2 µM.

In some embodiments, the GSK-3 inhibitor is present in the composition, kit, medium or culture of the present invention in an amount or concentration sufficient to induce totipotency, alone or in combination with other small molecule reprogramming agents. In some embodiments, the GSK-3 inhibitor is present in the composition, kit, medium or culture of the present invention at a concentration of 0.01, 0.05, 0.1, 0.2, 0.3, 0.4, 0.5, 0.6, 0.7, 0.8, 0.9, 1.0, 1.1, 1.2, 1.4, 1.6, 1.8, 2.0, 2.2, 2.4, 2.6, 2.8, 3.0, 3.2, 3.4, 3.6, 3.8, 4.0, 4.2, 4.4, 4.6, 4.8, 5.0, 5.2, 5.4, 5.6, 5.8, 6.0, 6.2, 6.4, 6.6, 6.8, 7.0, 7.2, 7.4, 7.6, 7.8, 8.0, 8.2, 8.4, 8.6, 8.8, 9.0, 9.2, 9.4, 9.6, 9.8, 10.0 µM or higher or a range consisting of any two of the foregoing values. Preferably, the GSK-3 inhibitor is present in the composition, kit, medium or culture of the present invention at a concentration of 0.5-10.0 µM, preferably 2.0-3 .0 µM, more preferably 2.5 µM. Most preferably, in one embodiment, the 1-Azakenpaullone is present in the composition, kit, medium or culture of the present invention at a concentration of 2.5 µM.

In some embodiments, the IKK signaling pathway inhibitor is present in the composition, kit, medium or culture of the present invention in an amount or concentration sufficient to induce pluripotency, alone or in combination with other small molecule reprogramming agents. In some embodiments, the IKK signaling pathway inhibitor is present in the composition, kit, medium or culture of the present invention at a concentration of 0.01, 0.05, 0.1, 0.2, 0.3, 0.4, 0.5, 0.6, 0.7, 0.8, 0.9, 1.0, 1.1, 1.2, 1.4, 1.6, 1.8, 2.0, 2.2, 2.4, 2.6, 2.8, 3.0, 3.2, 3.4, 3.6, 3.8, 4.0, 4.2, 4.4, 4.6, 4.8, 5.0, 5.2, 5.4, 5.6, 5.8, 6.0, 6.2, 6.4, 6.6, 6.8, 7.0, 7.2, 7.4, 7.6, 7.8, 8.0, 8.2, 8.4, 8.6, 8.8, 9.0, 9.2, 9.4, 9.6, 9.8, 10.0 µM or higher or a range consisting of any two of the foregoing values. Preferably, the IKK signaling pathway inhibitor is present in the composition, kit, medium or culture of the present invention at a concentration of 0.1-10.0 µM, preferably 0.3-1 µM, more preferably 0.5 µM. Most preferably, in one embodiment, the WS6 is present in the composition, kit, medium or culture of the present invention at a concentration of 0.5 µM.

In some embodiments, the HDAC inhibitor is present in the composition, kit, medium or culture of the present invention in an amount or concentration sufficient to induce totipotency, alone or in combination with other small molecule reprogramming agents. In some embodiments, the HDAC inhibitor is present in the composition, kit, medium or culture of the present invention at a concentration of 0.01, 0.05, 0.1, 0.2, 0.3, 0.4, 0.5, 0.6, 0.7, 0.8, 0.9, 1.0, 1.1, 1.2, 1.4, 1.6, 1.8, 2.0, 2.2, 2.4, 2.6, 2.8, 3.0, 3.2, 3.4, 3.6, 3.8, 4.0, 4.2, 4.4, 4.6, 4.8, 5.0, 5.2, 5.4, 5.6, 5.8, 6.0, 6.2, 6.4, 6.6, 6.8, 7.0, 7.2, 7.4, 7.6, 7.8, 8.0, 8.2, 8.4, 8.6, 8.8, 9.0, 9.2, 9.4, 9.6, 9.8, 10.0 µM or higher or a range consisting of any two of the foregoing values.

In some embodiments, the histone methyltransferase inhibitor is present in the composition, kit, medium or culture of the present invention in an amount or concentration sufficient to induce totipotency, alone or in combination with other small molecule reprogramming agents. In some embodiments, the histone methyltransferase inhibitor is present in the composition, kit, medium or culture of the present invention at a concentration of 0.01, 0.05, 0.1, 0.2, 0.3, 0.4, 0.5, 0.6, 0.7, 0.8, 0.9, 1.0, 1.1, 1.2, 1.4, 1.6, 1.8, 2.0, 2.2, 2.4, 2.6, 2.8, 3.0, 3.2, 3.4, 3.6, 3.8, 4.0, 4.2, 4.4, 4.6, 4.8, 5.0, 5.2, 5.4, 5.6, 5.8, 6.0, 6.2, 6.4, 6.6, 6.8, 7.0, 7.2, 7.4, 7.6, 7.8, 8.0, 8.2, 8.4, 8.6, 8.8, 9.0, 9.2, 9.4, 9.6, 9.8, 10.0 µM or higher or a range consisting of any two of the foregoing values.

In some embodiments, the Src kinase inhibitor is present in the composition, kit, medium or culture of the present invention in an amount or concentration sufficient to induce pluripotency, alone or in combination with other small molecule reprogramming agents. In some embodiments, the Src kinase inhibitor is present in the composition, kit, medium or culture of the present invention at a concentration of 0.01, 0.05, 0.1, 0.2, 0.3, 0.4, 0.5, 0.6, 0.7, 0.8, 0.9, 1.0, 1.1, 1.2, 1.4, 1.6, 1.8, 2.0, 2.2, 2.4, 2.6, 2.8, 3.0, 3.2, 3.4, 3.6, 3.8, 4.0, 4.2, 4.4, 4.6, 4.8, 5.0, 5.2, 5.4, 5.6, 5.8, 6.0, 6.2, 6.4, 6.6, 6.8, 7.0, 7.2, 7.4, 7.6, 7.8, 8.0, 8.2, 8.4, 8.6, 8.8, 9.0, 9.2, 9.4, 9.6, 9.8, 10.0 µM or higher or a range consisting of any two of the foregoing values.

In some embodiments, the cAMP activator is present in the composition, kit, medium or culture of the present invention in an amount or concentration sufficient to induce totipotency, alone or in combination with other small molecule reprogramming agents. In some embodiments, the cAMP activator is present in the composition, kit, medium or culture of the present invention at a concentration of 0.01, 0.05, 0.1, 0.2, 0.3, 0.4, 0.5, 0.6, 0.7, 0.8, 0.9, 1.0, 1.1, 1.2, 1.4, 1.6, 1.8, 2.0, 2.2, 2.4, 2.6, 2.8, 3.0, 3.2, 3.4, 3.6, 3.8, 4.0, 4.2, 4.4, 4.6, 4.8, 5.0, 5.2, 5.4, 5.6, 5.8, 6.0, 6.2, 6.4, 6.6, 6.8, 7.0, 7.2, 7.4, 7.6, 7.8, 8.0, 8.2, 8.4, 8.6, 8.8, 9.0, 9.2, 9.4, 9.6, 9.8, 10.0 µM or higher or a range consisting of any two of the foregoing values.

In some embodiments, the cellular metabolism modulator is present in the composition, kit, medium or culture of the present invention in an amount or concentration sufficient to induce totipotency, alone or in combination with other small molecule reprogramming agents. In some embodiments, the cell metabolism modulator is present in the composition, kit, medium or culture of the present invention at a concentration of 0.01, 0.05, 0.1, 0.2, 0.3, 0.4, 0.5, 0.6, 0.7, 0.8, 0.9, 1.0, 1.1, 1.2, 1.4, 1.6, 1.8, 2.0, 2.2, 2.4, 2.6, 2.8, 3.0, 3.2, 3.4, 3.6, 3.8, 4.0, 4.2, 4.4, 4.6, 4.8, 5.0, 5.2, 5.4, 5.6, 5.8, 6.0, 6.2, 6.4, 6.6, 6.8, 7.0, 7.2, 7.4, 7.6, 7.8, 8.0, 8.2, 8.4, 8.6, 8.8, 9.0, 9.2, 9.4, 9.6, 9.8, 10.0 µM or higher or a range consisting of any two of the foregoing values.

In a particular embodiment, preferred combinations of the small molecule reprogramming reagents in the composition, kit, medium or culture of the present invention are listed in Table 1.

**Table 1. Preferred combinations of the small molecule reprogramming reagents in the composition, kit, medium or culture of the present invention**

| | |
|---|---|
| 1# | TTNPB |
| 2# | TTNPB, TSA, BIX01294 |
| 3# | TTNPB, VPA, A366 |
| 4# | TTNPB, TSA, BIX01294, Dasatinib |
| 5# | TTNPB, VPA, A366, Dasatinib |
| 6# | TTNPB, TSA, BIX01294, Dasatinib, AZD2858 |
| 7# | TTNPB, Dasatinib, AZD2858 |
| 8# | TTNPB, WS6, 1-Azakenpaullone |
| 9# | AZD2858, Forskolin, PGE2 |
| 10# | 1-Azakenpaullone, WS6, WH4-023, Forskolin, PGE2 |
| 11# | TTNPB, Dasatinib, AZD2858, JQ-1, CH5138303 |
| 12# | BMP6, Metformin, AZD1080, PGE2, WH4-023 |
| 13# | TTNPB, WS6, 1-Azakenpaullone, TGFb-2 |
| 14# | TTNPB, WS6, 1-Azakenpaullone, Metformin |
| 15# | TTNPB, WS6, 1-Azakenpaullone, Forskolin, PGE2 |
| 16# | TTNPB, WS6, 1-Azakenpaullone, PS48 |
| 17# | TTNPB, WS6, 1-Azakenpaullone, IndolactamV |
| 18# | TTNPB, Dasatinib, AZD2858, bpv |
| 19# | TTNPB, WS6, 1-Azakenpaullone, Dasatinib, AZD2858 |
| 20# | TTNPB, WS6, 1-Azakenpaullone, PBS |
| 21# | TTNPB, WS6, 1-Azakenpaullone, Quercetin |
| 22# | TTNPB, WS6, 1-Azakenpaullone, 2-DG |
| 23# | TTNPB, WS6, 1-Azakenpaullone, DMOG |

Preferably, in one embodiment, the composition, kit or use according to the present invention comprises or preferably consists of: RA signaling pathway activator, GSK-3 inhibitor and IKK signaling pathway inhibitor.

More preferably, in one embodiment, the RA signaling pathway activator is TTNPB, the GSK-3 inhibitor is 1-Azakenpaullone, and the IKK signaling pathway inhibitor is WS6.

Most preferably, in one embodiment, the RA signaling pathway activator is 0.2 µM TTNPB, the GSK-3 inhibitor is 2.5 µM 1-Azakenpaullone, and the IKK signaling pathway inhibitor is 0.5 µM WS6.

### E. Culture medium

In one aspect, the present invention provides a culture medium comprising the composition described herein. The composition comprises: (a) an RA signaling pathway activator; and (b) one or more of a GSK-3 inhibitor, an IKK signaling pathway inhibitor, an HDAC inhibitor, a histone methyltransferase inhibitor, a Src kinase inhibitor, a cAMP activator, and a cell metabolism modulator.

In some embodiments, the medium of the present invention comprises a basal medium. Exemplary basal media include, but are not limited to, DMEM, Knockout DMEM, RPMI 1640, and DMEM/F12. In some embodiments, the basal medium is selected from the group consisting of DMEM, Knockout DMEM, RPMI 1640, and DMEM/F12.

In a particular embodiment, the culture medium of the present invention comprises cytokines and/or growth factors. In a particular embodiment, the culture medium of the present invention is substantially free or free of cytokines and/or growth factors. In certain embodiments, the culture medium comprises one or more supplements including, but not limited to, serum, extracts, growth factors, hormones, cytokines, and the like.

In an illustrative embodiment, the culture medium comprises one or more of the following cytokines or growth factors: epidermal growth factor (EGF), acidic fibroblast growth factor (aFGF), basic fibroblast growth factor ( bFGF), leukemia inhibitory factor (LIF), hepatocyte growth factor (HGF), insulin-like growth factor-1 (IGF-1), insulin-like growth factor-2 (IGF-2), keratinocyte growth factor (KGF), nerve growth factor (NGF), platelet-derived growth factor (PDGF), transforming growth factor (iKTGF-β), vascular endothelial growth factor (VEGF), transferrin, various interleukins (such as IL-1 to E-18) , various colony-stimulating factors such as granulocyte/macrophage colony-stimulating factor (GM-CSF), various interferons such as IFN-γ, and other cytokines that have effects on stem cells such as stem cell factor (SCF) and erythropoietin (Epo). These cytokines are commercially available, e.g., from R&D Systems Minneapolis, Minn, and may be native or recombinant. In certain embodiments, the growth factors and cytokines may be added at concentrations contemplated herein for small molecule reprogramming agents.

Any suitable vessel or cell culture vessel may be used as a support for cell culture in basal medium and/or cell culture supplements. A matrix coating on the support is not necessary. However, coating the surface of the culture vessel with an anchorage-promoting matrix (e.g., collagen, fibronectin, RGD-containing polypeptide, gelatin, etc.) promotes the attachment of the cells and, in a particular embodiment, may enhance the effects of the media and the supplements disclosed herein. Suitable matrices for culturing and passaging cells are known in the art and include, but are not limited to, vitronectin, gelatin, laminin, fibronectin, collagen, elastin, osteopontin, a mixture of matrices produced by naturally occurring cell lines such as Matrigel^{™} and synthetic or artificial surfaces such as polyamine monolayers and carboxyl terminated monolayers.

### F. Cell production method

In one aspect, the present invention provides a method of producing induced totipotent stem cells, the method comprising culturing the cells in a medium as described herein, thereby producing the induced totipotent stem cells. In some embodiments, the culture medium comprises a composition described herein. In some embodiments, the composition comprises: (a) an RA signaling pathway activator; and (b) one or more of a GSK-3 inhibitor, an IKK signaling pathway inhibitor, an HDAC inhibitor, a histone methyltransferase inhibitor, a Src kinase inhibitor, a cAMP activator, and a cell metabolism modulator.

The cells used as the starting material for the method of the present invention may be a wide variety of cells as described herein. For example, the method of the present invention may start with pluripotent cells, e.g., pluripotent stem cells, such as induced totipotent stem cells, or the method of the present invention may start with non-pluripotent cells, e.g., non-pluripotent stem cells, such as somatic cells.

In some embodiments, the method of the present invention comprises culturing pluripotent stem cells in a medium as described herein, thereby producing the induced totipotent stem cells.

In some embodiments, the pluripotent stem cells are embryonic stem cells or induced pluripotent stem cells.

In some embodiments, the method of the present invention comprises culturing non-pluripotent stem cells in a medium as described herein, thereby producing the induced totipotent stem cells.

In some embodiments, the method comprises reprogramming non-pluripotent cells to pluripotent stem cells.

In some embodiments, the non-pluripotent cells are selected from the group consisting of somatic cells and/or adult stem cells.

In some embodiments, the reprogramming of a non-pluripotent cell to a pluripotent stem cell comprises expressing one or more reprogramming factors selected from the group consisting of Oct4, Sox2, Klf4 and c-Myc in the non-pluripotent cells.

In some embodiments, the culturing of the pluripotent stem cells lasts for 1, 2, 3, 4, 5, 6, 7, 8, 9, 10, 11, 12, 13, 14, 15, 16, 17, 18, 19 or 20 days.

### G. Culture

In one aspect, the present invention provides a culture comprising the culture medium described herein and pluripotent stem cells. In some embodiments, the culture medium comprises the composition described herein. In some embodiments, the composition comprises: (a) an activator of RA signaling pathway; and (b) one or more of a GSK-3 inhibitor, an IKK signaling pathway inhibitor, an HDAC inhibitor, a histone methyltransferase inhibitor, a Src kinase inhibitor, a cAMP activator, and a cell metabolism modulator.

The cells comprised in the cultures described herein can be a variety of cells as described herein. For example, the cells may be initial cells for the culture or induction described herein, for examples, pluripotent cells, e.g., pluripotent stem cells such as iPSCs, or non-pluripotent cells, e.g., non-pluripotent stem cells such as somatic cells. Alternatively, the cells may be intermediate or final cells cultured or induced as described herein. The intermediate cells may be cells with a variety of developmental potentials that differ from the initial and final cells. The final cells may be totipotent stem cells as described herein.

In some embodiments, the culture according to the present invention comprises the culture medium as described herein and pluripotent stem cells.

In some embodiments, the pluripotent stem cells are embryonic stem cells or induced pluripotent stem cells.

In some embodiments, the culture according to the present invention comprises the culture medium as described herein and totipotent stem cells.

In some embodiments, the totipotent stem cells are induced totipotent stem cells.

Preferably, in some embodiments, the totipotent stem cells can be produced by the methods described herein.

### H. Totipotent cells and the characterization thereof

Recently, in a research report published in the international journal *Nature Cell Biology* (Posfai, E. et al. Evaluating totipotency using criteria of increasing stringency. Nature Cell Biology 23, 49-60, doi: 10.1038/s41556-020-00609-2 (2021)), the scientists from institutions such as Karolinska Institutet, Sweden have defined a golden standard for distinguishing between pluripotent stem cells and totipotent stem cells.

The research indicates that in nature, mammalian totipotent cells exist only in the embryos of early development stages. Correspondingly, for mice, only zygotes and 2-cell-stage blastomeres have totipotency, which is gradually lost as the embryos develop. The strictest definition of totipotency means that a cell can develop into an entire embryo or organism. A broader definition of totipotency means that the cell has a bidirectional development potential to both intra-embryonic and extra-embryonic cell types. So far, scientists have created mouse stem cell lines in pluripotent state. However, these cells can only develop into cells of embryonic components and do not have the potential to develop into extra-embryonic cell types.

In this study, the researchers set a criterion for assessing whether cells are truly totipotent through a combination study. Together, they identified four criteria for totipotency in mouse stem cell lines: 1) it is required that the transcriptomic properties or gene expression profiles of these cells are more similar to those of early totipotent embryos rather than later-stage embryos; 2) they can differentiate *in vitro* into the extra-embryonic cell lineages and in turn into extra-embryonic cell types; 3) these cells can form blastoids through induced development *in vitro,* and can simulate some early embryonic development events; and 4) these cells can participate in intra-embryonic and extra-embryonic differentiation and differentiate into cell types that normally express the corresponding genetic markers (also known as embryonic-extra-embryonic chimerism) when injected into early mouse embryos. The researchers then tested two mouse stem cell lines (Extended Pluripotent Stem Cells: L-EPSC and D-EPSC) previously reported to possess potential totipotency and evaluated them using these golden standards, finding that neither of them met the criteria for totipotent stem cells.

The totipotent stem cells induced and cultured by the inventors exhibit 1) the transcriptome characteristics similar to those of mouse embryo zygotes and 2-cell blastomeres, and are transformed into the levels similar to those of totipotent cells *in vivo* in terms of chromatin accessibility, DNA methylation level and cell metabolism mode; 2) as demonstrated by the direct differentiation of monolayer cells *in vitro,* embryoid body differentiation in suspension and teratoma differentiation *in vivo* assays, the totipotent stem cells induced and cultured by the inventors have the ability to differentiate into extra-embryonic cells which is not possessed by pluripotent stem cells. An *in vivo* chimerism experiment also strongly prove that the totipotent stem cells induced and cultured by the inventor have high-efficiency bidirectional development potential to both intra-embryonic and extra-embryonic tissues; 3) more importantly, the induced totipotent stem cells can be independently induced and developed into mouse blastocysts *in vitro,* and correctly express mouse blastocyst marker genes. The induced blastocyst can show a series of characteristics after embryo implantation when continuously culturing *in vitro,* and the induced blastocyst can also be implanted into the uterus for continue development after being transplanted into mice. Therefore, the induced mouse totipotent stem cells have the potential to independently develop into an intact living body, but not through a traditional sperm-oocyte binding process.

In one aspect, the present invention provides an induced totipotent stem cell that can be produced by the method described herein. In some embodiments, the method comprises culturing cells in the culture medium described herein, thereby producing the induced totipotent stem cells. In some embodiments, the culture medium comprises the composition described herein. In some embodiments, the composition comprises: (a) an activator of RA signaling pathway; and (b) one or more of a GSK-3 inhibitor, an IKK signaling pathway inhibitor, an HDAC inhibitor, a histone methyltransferase inhibitor, a Src kinase inhibitor, a cAMP activator, and a cell metabolism modulator.

The induced totipotent stem cells produced using the culture platform described herein can be characterized in a variety of ways.

The transcription of classical marker genes and repeats (e.g., MERVL, Zscan4, ZFP352, Tcstv3 and Gm6763) of totipotency in the obtained induced totipotent stem cells may be detected by RT-qPCR reaction. Comparing with the initial cells such as embryonic stem cells (mESCs), the induced totipotent stem cells according to the present invention can exhibit high expression of totipotency marker genes and repeats (e.g., MuERVL, Zscan4, ZFP352, Tstv3 and Gm6763), meaning that pluripotent embryonic stem cells undergo a cell fate transition to totipotent stem cells. In some embodiments, the expression level of one or more of the totipotency marker genes and repeats in the induced totipotent stem cell is 1, 2, 3, 4, 5, 6, 7, 8, 9, 10, 15, 20, 25, 30, 35, 40, 45, 50, 55, 60, 65, 70, 75, 80, 85, 90, 95, 100, 150 , 200, 250, 300, 350, 400, 450, 500, 550, 600, 650, 700, 750, 800, 850, 900, 950, 1000 or more compared to the initial cell times, or within a range consisting of any two of the foregoing values.

In order to further explore the molecular biological characteristics of induced totipotent stem cells, transcriptome sequencing (RNA-seq) and single-cell RNA sequencing (scRNA-seq) can also be used to analyze the changes in the transcriptional level in initial cells such as embryonic stem cells after they have acquired totipotency. This may comprise the analysis of the enrichment (GSEA analysis) of totipotency marker genes and pluripotency marker genes in the initial cells such as embryonic stem cells and induced totipotent stem cells. It can be found that totipotency marker genes, for example their specific expression in 2-cell-stage embryo, are significantly enriched in the induced totipotent stem cells according to the present invention, while pluripotency marker genes are significantly enriched in the initial cells such as embryonic stem cells.

Cluster analysis can be used to analyze the similarity of the whole transcriptome level in the initial cells such as embryonic stem cells and in the induced totipotent stem cells according to the present invention at various stages of embryonic development. It can be found that the induced totipotent stem cells according to the present invention are close to totipotent 1-cell-stage and 2-cell-stage embryos at the whole transcriptome level, and thus are in a totipotent state at the transcriptional level.

It can be confirmed through a principal component analysis (PCA) of whole transcriptome that the induced totipotent stem cells according to the present invention may be between the totipotent 1-cell-stage and 2-cell-stage embryos in terms of developmental stage, while the initial cells such as embryonic stem cells are closer to blastocysts at a later developmental stage.

By analyzing the enrichment of gene sets in the induced totipotent stem cells according to the invention compared to initial cells such as embryonic stem cells for the specific expression in zygote, 2-cell stage, 4-cell stage, 8-cell stage and 16-cell stage of embryonic development, totipotency marker gene sets specifically expressed in zygotes and the 2-cell stage can be significantly enriched in the induced totipotent stem cells according to the present invention.

UMAP analysis can be performed on the single-cell RNA sequencing (scRNA-seq) results of the induced totipotent stem cells according to the present invention and control cells. The induced totipotent stem cell according to the present invention may closely resemble 2-cell embryos with totipotency and exhibit high expression of totipotency marker genes and low expression of pluripotency marker genes, having totipotent transcriptome signatures.

In order to explore the chromatin accessibility characteristics of the induced totipotent stem cells according to the present invention, the induced totipotent stem cells according to the present invention can be analyzed by the Assay for Transposase-Accessible Chromatin using sequencing (ATAC-seq). Chromatin accessibility indicates the activation or repression of genes. The induced totipotent stem cells according to the present invention may show a closed peak and an open peak of 5 kb near the transcription start site (TSS), an open or closed state similar to the 2-cell embryonic (2C) stage. In addition, compared with the initial cells such as embryonic stem cells, the induced totipotent stem cells according to the present invention may show higher openness in the vicinity of some important totipotent genes and reverse transcription elements such as Zscan4c, Zscan4d, Zscan4f, ZFP352, MERVL, etc. while the classical pluripotency genes such as POU5f1, ZFP42, NANOG, KLF4, ESRRB, etc may be in a closed state. This means that the induced totipotent stem cells according to the present invention may have chromatin accessibility similar to that of 2-cell stage embryos with totipotency.

In order to detect the genomic methylation level of induced totipotent stem cells according to the present invention, the reduced representation bisulfite sequencing (RRBS) can be used to characterize the DNA methylome of the induced totipotent stem cells according to the present invention. Through the global methylation principal component analysis of the RRBS sequencing results, it can be seen that the induced totipotent stem cells according to the present invention may be closer to the 1-cell-stage and 2-cell-stage embryos with totipotency in terms of developmental stage than the initial cells such as embryonic stem cells.

Pluripotent stem cells do not have the capacity to differentiate into extra-embryonic cells. In order to verify the ability of the induced totipotent stem cells according to the present invention to differentiate into extra-embryonic cell types, commercial trophoblast stem cells (TSCs) can be used as a positive control to detect the expression of the specific protein of trophectoderm stem cells (CDX2) by immunofluorescence staining. CDX2 is a classic specific gene of mouse trophectoderm stem cell. By immunofluorescence detection of its protein expression level, combined with the change of pluripotent stem cell marker Oct4, it can be judged whether the differentiation to trophectoderm stem cell lineage has occurred. The induced totipotent stem cells according to the present invention can be efficiently induced to significantly express CDX2 while the expression of Oct4 is down-regulated, and thus have the capacity to differentiate into extra-embryonic parts, i.e., trophectoderm stem cells.

The transcription of trophectoderm stem cell-specific genes during the induction of the induced totipotent stem cells according to the present invention into trophectoderm stem cells can be analyzed by RT-qPCR. The induced totipotent stem cells according to the present invention may gradually increase trophectoderm stem cell specific marker genes during the induction process, including but not limited to CDX2, Elf5, TFAP2C and Esx1, which is similar to the trophoblast stem cells (TSCs) as the positive control.

CDX2 is a classic specific gene of mouse trophectoderm stem cell. The ability of the induced totipotent stem cells according to the present invention to differentiate into embryoid bodies can be determined by detecting CDX2-positive cells (representive of extra-embryonic cells, indicating the differentiation of the totipotent stem cells into extra-embryonic cell types) via immunofluorescence staining, thereby determining the ability of the induced totipotent stem cells according to the present invention differentiating into embryoid bodies. Embryoid bodies differentiation experiments demonstrate that the induced totipotent stem cells according to the present invention possess the capacity to differentiate into extra-embryonic parts, i.e., trophectoderm stem cells, *in vitro,* which is not possessed by pluripotent stem cells.

The teratoma assay is a classic experiment to test the ability of cells to randomly differentiate into the three germ layers and extra-embryonic lineages. The capacity of the induced totipotent stem cells according to the present invention to differentiate into teratomas can be determined by observing the tissue section under a microscope to find the specific tissue structure of three germ layers and extra-embryonic lineages. The teratoma differentiation experiment demonstrates that the induced totipotent stem cells according to the present invention possess the capacity to differentiate into extra-embryonic parts *in vitro,* which is not possessed by pluripotent stem cells.

Chimeric embryo development experiment can be used to demonstrate that induced totipotent stem cells according to the present invention have the developmental potential to differentiate into both intra-embryonic and extra-embryonic cell types. Blastocysts from Rosa26-tdTomato mice can be used to establish fluorescent cell lines of mouse induced totipotent stem cells or mouse embryonic stem cells (mESCs) for injecting into 8-cell stage embryos, resulting in stem cells stably expressing tdTomato fluorescence. If the stem cells injected into the embryo develop with the embryo and differentiate into intra-embryonic and extra-embryonic parts, fluorescence can be observed in the corresponding intra-embryonic and extra-embryonic parts. In a chimeric embryos *in vivo* development experiments of induced totipotent stem cell (e.g., development to E4.5), the induced totipotent stem cells according to the present invention can be embedded into both the inner cell mass (ICM) within the embryo and the trophectoderm (TE) outside the embryo, as determined by chimerism of tdTomato positive cells. Differentiation into extra-embryonic cell types can also be further confirmed by co-localization of tdtomato fluorescence and CDX2 fluorescence. In the chimeric embryos *in vivo* development experiments of the induced totipotent stem cells according to the present invention (e.g. development to E4.5, E7.5 and E12.5), co-localization of tdtomato fluorescence and CDX2 fluorescence can be observed. It was confirmed that the induced totipotent stem cells according to the present invention can participate in the development of the trophectoderm (TE) and have the capacity to differentiate into extra-embryonic parts.

For further development of chimeric embryos (e.g., E7.5), the capacity of the induced totipotent stem cells according to the present invention to differentiate towards the extra-embryonic parts can also be confirmed by immunofluorescence staining of the Oct4, a classic marker of embryonic ectoderm (EPI), and ELF5, a classic marker of placental cone (EPC) and extra-embryonic ectoderm (ExE). The induced totipotent stem cells according to the present invention can be embedded into the embryonic embryonic epiblast (EPI), extra-embryonic placental cone (EPC) and extra-embryonic ectoderm (ExE). It was demonstrated that the induced totipotent stem cells according to the present invention can still have the capacity to participate in the development of extra-embryonic tissues of the embryo after embryo implantation.

For further development of chimeric embryos (e.g., E12.5), it can also be confirmed by analyzing the chimerism of tdTomato-positive cells that the induced totipotent stem cells according to the present invention may be embedded into embryos (Em), extra-embryonic tissues placenta (Pl) and yolk sac (Yo). It was demonstrated that the induced totipotent stem cells according to the present invention can still have the capacity to participate in the development of extra-embryonic tissues of the embryo after embryo implantation.

The capacity of the induced totipotent stem cells according to the present invention to independently develop into blastocysts can be examined under the culture conditions used to induce blastocysts. The blastocysts induced from the induced totipotent stem cells according to the present invention can have the morphological characteristics highly similar to normal blastocysts. The blastocysts induced from the induced totipotent stem cells according to the present invention may have the three cell lineages of normal blastocysts *in vivo* as confirmed by immunofluorescence staining, demonstrating that the induced totipotent stem cells according to the present invention can be efficiently induced into blastocysts with correct structure and correct gene expression.

The blastocysts induced from the induced totipotent stem cells according to the present invention can be further cultured *in vitro* to generate three-dimensional structures similar to post-implantation embryos (e.g., E4.5-E5.5 stages), comprising ectoderm (staining positive for TFAP2C) and EPI (staining positive for Oct4) surrounded by endoderm (staining positive for SOX17) as two hemispheres.

The blastocysts induced from the induced totipotent stem cells according to the present invention can be implanted in a uterus for further development *in vivo,* and can trigger a decidualization reaction after implantation in the uterus and continue to grow.

As discussed above, the induced totipotent stem cells according to the present invention may be characterized by one or more of the above features. Such characterization can be performed using the methods described herein or well known to those skilled in the art.

### I. Application

The induced totipotent stem cells according to the present invention can be used for a wide range of desired applications in research, industry and clinic. For example, various products can be produced via differentiation of the induced totipotent stem cells of the present invention, which can be used, for example, to construct models, study targets, develop surrogates, and for other potential therapeutic or diagnostic applications.

The induced totipotent stem cells according to the present invention can be used to induce the production of organisms. The organisms can be used for potential scientific, therapeutic and diagnostic applications such as the construction of disease models. In one aspect, the present invention provides an organism derived from the induced totipotent stem cells described herein. The organism may be a eukaryotic organism, including but not limited to animals, plants, fungi, and other eukaryotic organisms known in the art. The animals may include, but are not limited to, mammals, e.g., primates such as humans, non-human primates, non-primates, bovines, equines, ovines, porcines, canines, lagomorphs, rodents, such as monkeys, cows, sheep, pigs, dogs, rabbits, rats or mice. Preferably, in some embodiments, the organism is a rodent or mammal. In some embodiments, the mammal is not a human.

The plant may be a monocotyledonous or dicotyledonous plant, or be a crop or grain plant, such as cassava, corn, sorghum, soybean, wheat, oat or rice. The plant may also be algae, trees or a yielder plants, fruits or vegetables (for example, trees such as citrus trees, e.g. orange, grapefruit or lemon trees; peach or nectarine trees; apple or pear trees; nut trees such as almond or walnut or pistachio trees; plants of *Solanum* genus; plants of *Brassica* genus; plants of *Lactuca* genus; plants of *Spinacia* genus; plants of *Capsicum* genus; cotton, tobacco, asparagus, carrots, cabbage, broccoli, cauliflower, tomato, eggplant, pepper, lettuce, spinach, strawberries, blueberries, raspberries, blackberries, grapes, coffee, cocoa, etc.).

The induced totipotent stem cells according to the present invention may be used to induce the production of organoids. The organoids can be used for constructing a disease model, transplantation therapy, or other potential scientific, therapeutic and diagnostic applications. In one aspect, the present invention provides an organoid derived from the induced totipotent stem cells described herein. The organoid may be an organoid from, but not limited to, the following organs:
Musculoskeletal system, including the human skeleton, such as bones, carpals, clavicle, femur, fibula, humerus, jaw, metacarpal, metatarsal, ossicles, patella, phalanges, radius, skull, tarsus, tibia, ulna, ribs, spine, pelvis, sternum, cartilage; joints such as fibrous joint, cartilaginous joint and synovial joint; muscular systems such as muscles, tendons, diaphragms;
Circulation system, including cardiovascular system, such as peripheral blood supply system (arteries, veins, lymphatics), heart; the lymphatic system, primary (bone marrow, thymus), secondary (spleen, lymph nodes), glymphatic system;
Nervous system, including brain, such as hindbrain (medulla oblongata, pons, cerebellum), midbrain, forebrain (diencephalon (retina, optic nerve), cerebrum, limbic system), spinal cord, nerves, sensory system (ear, eye);
Epidermal system, including skin, subcutaneous tissues, breast (mammary gland);
Immune system, including myeloid cells, lymphocytes;
Respiratory system, including upper respiratory tract (nose, pharynx, larynx), lower respiratory tract (trachea, bronchi, lungs);
Digestive system, including mouth (salivary glands, tongue), upper gastrointestinal tract (oropharynx, laryngopharynx, esophagus, stomach), lower gastrointestinal tract (small intestine, appendix, large intestine, rectum, anus), affiliated digestive glands (liver, bile duct, pancreas);
Urinary system, including genitourinary system, kidneys, ureters, bladder, urethra;
Reproductive system, including female reproductive system (uterus, vagina, vulva, ovary, placenta), male reproductive system (scrotum, penis, prostate, testes, seminal vesicles);
Endocrine system, including pituitary, pineal, thyroid, parathyroid, adrenal, pancreatic islets.

The induced totipotent stem cells according to the present invention can be used to induce the production of tissues. The tissue can be used for construction of a disease model, transplantation therapy, or other potential scientific, therapeutic and diagnostic applications. In one aspect, the present invention provides a tissue derived from the induced totipotent stem cells described herein. The tissue includes, but is not limited to, an animal tissue and a plant tissue. The animal tissue includes, but is not limited to, an epithelial tissue, a muscle tissue, a neural tissue, and a connective tissue. The connective tissue includes, but is not limited to, connective tissue proper, including loose connective tissue (areolar tissue), dense connective tissue, adipose tissue, reticular connective tissue, elastic connective tissue; bone or cartilage tissue; blood; and lymph. Blood is the liquid tissue circulating in the cardiovascular system. Blood is composed of plasma and a variety of blood cells. Lymph is the fluid that flows in the lymphatic vessels, and is formed by the flow of tissue fluid into the lymph. Lymph eventually affluxs into veins. Lymph contains lymphocytes, and the composition of lymph varies under different physiological conditions. Preferably, in some embodiments, the tissue is blood.

The induced totipotent stem cells according to the present invention can be used to induce the production of differentiated cells. The differentiated cells can be used for the construction of a disease model, transplantation therapy, or other potential scientific, therapeutic and diagnostic applications. In one aspect, the invention provides a differentiated cell which is differentiated from the induced totipotent stem cell described herein. The differentiated cells may be cells from any organ or tissue previously described. Preferably, in some embodiments, the cells are immune cells, more preferably T cells or NK cells. Preferably, in some embodiments, the cells are neural cells, more preferably neurons or glial cells. Preferably, in some embodiments, the cells are blood cells, more preferably erythrocytes or white blood cells.

All publications, patent applications and patents mentioned in this specification are herein incorporated in their entirety by reference, to the same extent as if each individual publication, patent application or patent is specifically and individually indicated to be incorporated herein by reference.

Although the invention has been described in some detail by way of embodiments and examples for the purpose of clear understanding, it is obvious to those skilled in the art that certain changes and modifications can be made thereto in accordance with the teachings of the present invention without departing from the spirit or scope of the claims. The following Examples are provided for demonstrations rather than limitations. Those skilled in the art readily recognize that various non-critical parameters can be varied or modified to achieve substantially similar results.

### Example 1: Induction and preliminary identification of mouse induced totipotent stem cells

The inventors have surprisingly found that mouse pluripotent stem cells, after being treated with small molecule reprogramming reagents, have the characteristics of totipotent cells that are highly similar to mouse fertilized eggs or 2-cell stage embryonic cells. Such stem cells are referred to as mouse induced totipotent stem cells by the inventors.

### 1.1 Induction of mouse totipotent stem cells

When mouse pluripotent embryonic stem cells or mouse induced pluripotent stem cells (both commercially available or established by standardized experiments) are grown in a standard medium to a nearly 70% dish density, the cells were digested with 0.05% (v/v) trypsin to obtain single cells in suspension, and it was passaged at a ratio of about 1:10 (1 generation is enough) and seeded into the totipotent stem cell medium (the commercial mouse pluripotent stem cell basal medium was additionally supplemented with small molecule reprogramming reagents).

The commercial pluripotent stem cell basal medium used herein comprises: Knockout DMEM basal medium, and 5% KSR, 1% N2, 0.2% chemically defined lipid concentrate (CDL), 1% GlutaMAX^{™} (L-glutamine substitute), 1% dual antibiotics (penicillin/streptomycin), 1% non-essential amino acids, 0.1 mM β-mercaptoethanol, 50 ng/ml sodium L-ascorbyl-2-phosphate and 1000 U/mL mouse leukemia inhibitory factor (mLIF) (Yang, Y. et al. Derivation of pluripotent stem cells with in vivo intra-embryonic and extra-embryonic potency. Cell 169, 243-257. e225 (2017).).

The inventors tested various combinations of small molecule reprogramming agents to determine their effects on the induction of totipotent stem cells. Various small molecule reprogramming reagents are as described in the DETAILED DESCRIPTION OF THE INVENTION section herein. Some of the combinations tested are listed in Table 1. The inventors tested the performance of various aspects of the combinations, including the induction ratio of the totipotent stem cells, i.e., the 2C:tdTomato cell ratio, as shown in Figure 9.2. Some of these small molecule reprogramming reagents were also individually replaced and tested for performance, as shown in Figure 9.4. It should be noted that the induction ratio of the totipotent stem cells is not the only factor for evaluating the quality of the combinations. After evaluating the performance in various aspects, the inventors selected the optimal combination, namely the combination of 3 small molecule compounds, TTNPB, 1-Azakenpaullone and WS6, for use in other experiments. 2.5 µM 1-Azakenpaullone, 0.5 µM WS6, and 0.2 ₁₁M TTNPB were added to the aforementioned commercial pluripotent stem cell basal media to obtain the totipotent stem cell media used below.

The inventors also tested various basal media (including DEME, Knockout DMEM, RPMI 1640 and DMEM/F12) to determine whether their type had an effect on the induction of totipotent stem cells. As shown in Figure 9.3, there was no significant difference in the 2C::td (2C:tdTomato) and OCT4 fluorescence assays. As such, the basal medium type has no substantial effect on the induction of totipotent stem cells as described herein. In other experiments, Knockout DMEM was used.

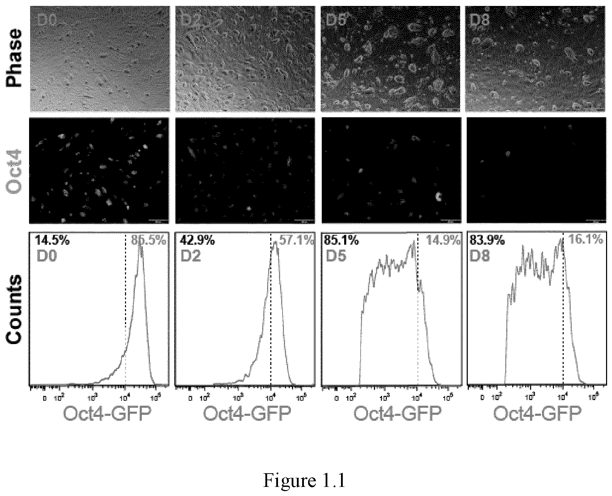

About two days after seeding in the totipotent stem cell media, mouse pluripotent embryonic stem cells gradually acquired mouse totipotent stem cell properties, which are characterized below. When the growth density of mouse totipotent stem cells was close to 70%, they were digested with 0.05% trypsin and subcultured at a ratio of 1:3-1:5. The expression of the pluripotency gene marker Oct4 in the resulting mouse totipotent stem cells was down-regulated, and the mouse totipotent stem cells could be maintained in subculture for more than 10 generations without losing their totipotency while maintaining a good clonal morphology (Figure 1.1)..

To investigate whether Dux and p53 are required for totipotent stem cell (ciTotiSC) induction, the inventors knocked out either Dux or p53 in mESCs. As expected, deletion of Dux significantly reduced the proportion of MERVL+ cells in mESCs (Figure 9.1a), with little increase in TAW-induced MERVL+ cells (Figure 9.1b). In Dux knockout cells, TAW consistently failed to induce totipotency genes such as Dux, Zscan4, Zfp352 and Tcstv3 (Figure 9.1c). These results demonstrate that Dux is essential for the induction of MERVL+ ciTotiSCs. To a lesser extent, p53 knockout also resulted in a reduction in the percentage of MERVL+ cells in TAW-treated or non-TAW-treated mESCs (Figures 9.1a and 9.1b). Further analysis showed that p53 knockout also reduced the expression of totipotency marker genes MERVL, Dux, Zscan4, Zfp352 and Tcstv3 compared with TAW-induced WT cells (Figure 9.1d). Together, these evidences suggest that induction of totipotent stem cells (ciTotiSCs) is partially dependent on p53.

### 1.2 Analysis of transcription of totipotency marker genes in mouse ciTotiSCs by RT-PCR

1) RT-qPCR reaction was performed using iQTM SYBR Green Supermix to detect the transcription of the classical totipotency marker genes and repeat sequences (MERVL, Zscan4, ZFP352, Tcstv3, Gm6763) of the resulting mouse ciTotiSCs. RT-qPCR reactions were performed in a Bio-Rad CFX384 Real-Time PCR System. Data results were analyzed and graphed in Prism 8 software.
2) qPCR reaction system

| Component | Volume (µl) |
|---|---|
| iQTM SYBR^{®} Green supermix (2x) | 5 |
| Forward primer (10 pmol) | 1 |
| Reverse primer (10 pmol) | 1 |
| cDNA template | 0.5 |
| Deionized water | 2.5 |
| Total volume | 10 |

3) qPCR primer sequences:

| | |
|---|---|
| MuERVL Forward primer | ATCTCCTGGCACCTGGTATG |
| MuERVL Reverse primer | AGAAGAAGGCATTTGCCAGA |
| Zscan4 Forward primer | GAGATTCATGGAGAGTCTGACTGATGAGT G |
| Zscan4 Reverse primer | GCTGTTGTTTCAAAAGCTTGATGACTTC |
| Zfp352 Forward primer | AAGGTCCCACATCTGAAGAA |
| Zfp352 Reverse primer | GGGTATGAGGATTCACCCA |
| Tcstv3 Forward primer | ACCAGCTGAAACATCCATCC |
| Tcstv3 Reverse primer | CCATGGATCCCTGAAGGTAA |
| Gm6763 Forward primer | CTGGTGGGAAGCTCTTCTTG |
| Gm6763 Reverse primer | TCAACGTTCCAAATTCAGCA |
| GAPDH Forward primer | CATGGCCTTCCGTGTTCCTA |
| GAPDH Reverse primer | GCCTGCTTCACCACCTTCTT |

4) qPCR reaction program

| Step | Temperature | Time/Cycle | Cycle Number |
|---|---|---|---|
| DNA polymerase activation and template denaturation | 95°C | 3 min | 1 |
| Denaturation | 95°C | 10 s | 40 |
| Annealing/Extens ion | 60°C | 30 s | |
| Melting Curve Analysis | | 55-95°C, increase 0.5°C per cycle, 5 seconds per step | |

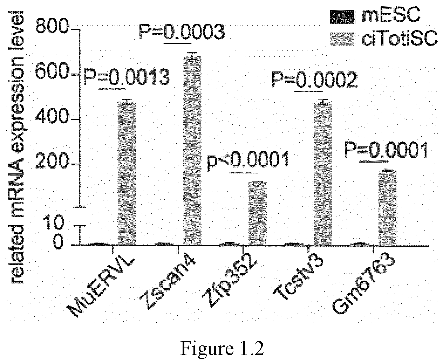

RT-qPCR analysis showed that compared with mouse pluripotent embryonic stem cells (mESCs) as starting cells, mouse ciTotiSCs showed significantly higher expression of totipotency marker genes and repeat sequences (MuERVL, Zscan4, ZFP352, Tstv3, Gm6763), which meant that mouse pluripotent embryonic stem cells underwent a cell fate transition to mouse totipotent stem cells (Figure 1.2).

### Example 2: Molecular characterization of mouse ciTotiSCs: transcriptome characterization of mouse ciTotiSCs by RNA-seq and scRNA-seq

To further explore the molecular biological characteristics of mouse induced pluripotent stem cells, the inventors used transcriptome sequencing (RNA-seq) and single-cell RNA sequencing (scRNA-seq) to analyze the changes at the transcriptome level after mouse pluripotent embryonic stem cells acquired totipotency.

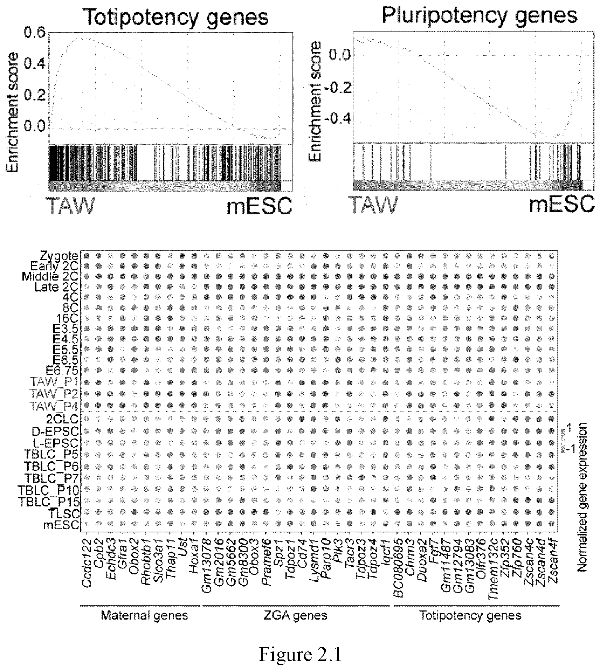

By analyzing the enrichment of 266 totipotency marker genes specifically expressed in mouse two-cell embryonic stage and 47 mouse pluripotency marker genes in mouse pluripotent embryonic stem cells (mESCs) and mouse ciTotiSCs (GSEA analysis), the inventors found that these 2-cell embryonic-stage-specific totipotency marker genes were significantly enriched in mouse ciTotiSCs (Figure 2.1, top left panel), while the mouse pluripotency marker genes were significantly enriched in mouse pluripotent embryonic stem cells (Figure 2.1 , top right panel). Using transcriptome sequencing (RNA-seq) analysis, it was found that the maternal gene, ZGA gene and totipotency gene were highly expressed in mouse totipotent stem cells, confirming that the cells were in a totipotent state (Figure 2.1, bottom panel).

Through clustering analysis of mouse pluripotent embryonic stem cells, mouse ciTotiSCs and the whole gene transcriptome levels of mouse embryonic development at various stages, the inventors found that mouse pluripotent embryonic stem cells were closer to the 3.5-day (E3.5) embryonic inner cell mass (ICM) of the pluripotent mouse at the level of the whole genome transcriptome, and therefore, exhibited a totipotent state at the transcriptional level; while mouse ciTotiSCs are closer to the 1- and 2-cell stage embryos of the totipotent mouse at the genome-wide transcriptome level, and therefore exhibited a totipotent state at the transcriptional level (Figure 2.2).

Through principal component analysis (PCA) of the whole genome transcriptome, the inventors further confirmed that mouse ciTotiSCs were between the mouse 1-cell and 2-cell stage embryos with totipotency, while mouse pluripotent embryonic stem cells (mESCs) and mouse expanded potential stem cells (EPSCs) were closer to mouse blastocysts at a later stage of development (Figure 2.3).

By analyzing the gene sets specifically expressed in fertilized egg, 2-cell stage, 4-cell stage, 8-cell stage and 16-cell stage of early embryonic development in mice, and the enrichment of these gene sets in mouse ciTotiSCs relative to mouse pluripotent embryonic stem cells, respectively, the inventors further confirmed that fertilized egg and 2-cell stage specific expression of totipotency marker gene sets were significantly enriched in mouse ciTotiSCs (Figure 2.4).

Through UMAP analysis of mouse ciTotiSCs, totipotent blastomere-like cells (TBLC, Shen, H. et al. Mouse totipotent stem cells captured and maintained through spliceosomal repression. Cell, doi:10.1016/j.cell.2021.04.020 (2021).), and single-cell RNA sequencing (scRNA-seq) results of normal mouse embryos at various stages (Deng, Q., Ramskold, D., Reinius, B. & Sandberg, R. Single-cell RNA-seq reveals dynamic, random monoallelic gene expression in mammalian cells. Science 343, 193-196 (2014).), it was found that, mouse ciTotiSCs were very close to totipotent 2-cell embryos in mice (TPSC and Late 2C shown in Figure 2.5), had high expression of totipotency marker genes and low expression of pluripotency marker genes, and had the characteristics of totipotency transcriptome; while totipotent blastomere-like cells (TBLC, Shen, H. et al. Mouse totipotent stem cells captured and maintained through spliceosomal repression. Cell, doi:10.1016/j.cell.2021.04.020 (2021).) were not close to totipotent mouse zygotes and 2-cell embryos. On the contrary, they were closer to the embryos at a later stage of development, E4.5-E5.5 days after the implantation (TBLC shown in Figure 2.5 and E4.5, E5.5). Moreover, the totipotency marker genes were not significantly activated, and the pluripotency marker genes were not effectively inhibited, so they could not be considered as totipotent cells (Figure 2.5).

### 2.2 Analysis of chromatin accessibility in mouse ciTotiSCs by ATAC-seq

To explore the chromatin accessibility characteristics in mouse ciTotiSCs (chromatin accessibility indicates the activation or repression of genes), the inventors used chromatin accessibility sequencing ATAC-seq (Assay for Transposase-Accessible Chromatin using sequencing) to analyze the chromatin accessibility of mouse ciTotiSCs, mouse pluripotent embryonic stem cells, mouse 2-cell stage embryos, and mouse blastocyst stage inner cell mass.

The results of the analysis showed that compared with mouse pluripotent embryonic stem cells, mouse ciTotiSCs had a 5kb closed peak and open peak near the transcription start site (TSS) with open or closed state similar to mouse 2-cell embryo (2C) stage; while mouse pluripotent embryonic stem cells (mESCs) were more similar to mouse blastocyst inner cell mass (ICM) (Figure 2.6). In addition, compared with mouse pluripotent embryonic stem cells, mouse ciTotiSCs showed higher levels of openness near some important pluripotency genes and retroelements, such as Zscan4c, Zscan4d, Zscan4f, ZFP352, MERVL, etc.; while the classical pluripotency genes, such as POU5f1, ZFP42, NANOG, KLF4, ESRRB, etc., were in a closed state (Figure 2.7).

These results indicate that mouse ciTotiSCs have chromatin accessibility similar to the in vivo totipotent 2-cell embryonic stage in mice.

### 2.3 Analysis of genomic methylation levels of mouse ciTotiSCs by RRBS

In order to detect the genomic methylation levels of mouse ciTotiSCs, the inventors used RRBS (Reduced Representation Bisulfite Sequencing) to characterize the genomic methylation levels of mouse ciTotiSCs and mouse pluripotent embryonic stem cells.

The inventors found that the overall methylation level of mouse pluripotent embryonic stem cells was 23.9%, which was close to the E6.5-E7.5 period after mouse embryo implantation (the overall methylation levels were 23.2% and 26.6%, respectively); while the methylation level of mouse ciTotiSCs was significantly reduced to 12.1%, similar to the fertilized egg and 2-cell and 4-cell stages of mouse embryos before implantation (overall methylation levels were 15.4%, 13.2% and 14.8%, respectively) (Figure 2.8).

By genome-wide methylation principal component (PCA) analysis of the RRBS sequencing results, the inventors reconfirmed that compared with mouse pluripotent embryonic stem cells, mouse ciTotiSCs were closer to mouse 1- and 2-cell stage embryos with totipotency in terms of developmental stage (Figure 2.9).

By further analyzing methylation levels near specific sites in the genome, it has been found that mouse ciTotiSCs and mouse totipotent embryos were in a state of hypomethylation for some totipotent repeat sequences, near the Zscan4 gene family and on the X chromosome. In contrast, neither mouse pluripotent embryonic stem cells nor mouse post-implantation E6.5-E7.5 embryos were found to have decreased methylation (Figure 2.10).

Since cells in the totipotent state have unique metabolic characteristics, in addition to the analysis of the transcriptome and epigenome, the inventors next determined the metabolome of the totipotent stem cells (ciTotiSCs). The inventors found that the metabolites with highest differences between totipotent stem cells (ciTotiSCs) and mESCs were very similar to those between totipotent 2C embryos and blastocysts (Figure 2.11, top). Further analysis showed that totipotent stem cells (ciTotiSCs) and 2C embryos were more likely to utilize one-carbon metabolism and reduced state related pathways for metabolism; while pluripotent mESCs and blastocysts had higher levels of purine metabolism and mitochondrial tricarboxylic acid (TCA) cycle metabolites, exhibiting a higher oxidation state (Figure 2.11, bottom).

### Example 3: Analysis of the ability of mouse ciTotiSCs to differentiate into trophoblast cells in vitro

Mouse pluripotent stem cells do not have the ability to differentiate into trophoblast cells. Figure 3.1 shows the schematic diagram of the experiment of differentiation of mouse ciTotiSCs, pluripotent embryonic stem cells (mESC, Ying, Q. L. et al. The ground state of embryonic stem cell self-renewal. Nature 453, 519-523, doi:10.1038/nature06968 (2008).) and pluripotent expanded potential stem cells (mEPS, Yang, Y. et al. Derivation of pluripotent stem cells with in vivo intra-embryonic and extra-embryonic potency. Cell 169, 243-257. e225 (2017).) into trophoblast stem cells in trophectoderm stem cell (TSC) media.

This experiment verified that the mouse ciTotiSCs of the present invention had the ability to differentiate into the extra-embryonic part, i.e., trophoblast stem cells, which the pluripotent stem cells did not have.

0.05% trypsin was used to digest mouse ciTotiSCs, embryonic stem cells (mESCs) and pluripotent expanded potential stem cells (mEPS) cultured on feeder cells (mouse embryonic fibroblasts, widely used in stem cell culture)). The digested cells were spread on a 0.3% gelatin-coated cell culture plate, and the suspended cells were collected after half an hour, and the feeder cells were adhered to the cell culture plate to remove the feeder cells.

Mouse ciTotiSCs, embryonic stem cells (mESCs) and pluripotent expanded potential stem cells (mEPS) from feeder-depleted cells were seeded in a mouse trophoblast stem cell medium (detailed below) at a density of 1 × 10⁵ cells per well of a 12-well plate. The medium was changed once a day. Cells were harvested on days 0, 4, and 8 for RT-qPCR to detect the transcription of mouse trophoblast stem cell specific genes (CDX2, Elf5, Tfap2c, Esx1; Figure 3.2), with commercial trophoblast stem cells (TSCs) as the positive control. Immunofluorescence staining was performed on day 12 to detect the expression of mouse trophectoderm cell specific protein (CDX2) (Figure 3.3).

Mouse trophoblast stem cell (TSC) medium (Posfai, E. et al. Evaluating totipotency using criteria of increasing stringency. Nature Cell Biology 23, 49-60, doi:10.1038/s41556-020-00609-2 (2021).): RPMI 1640 Basal Medium (Gibco, C11875500BT), 20% fetal bovine serum, 1X GlutaMAX (L-Glutamine substitute), 1% dual antibiotics (penicillin/streptomycin), 1% Nonessential amino acids, 1× pyruvate sodium (Gibco, 11360070), 1 mM β-mercaptoethanol, 25 ng/ml FGF4 (R&D systems, 235-F4) and 1 µg/ml heparin (Sigma-Aldrich, H3149).

### 3.1 Analysis of mouse trophoblast stem cell specific protein CDX2 expression by immunofluorescence staining

CDX2 is a classic mouse trophoblast stem cell specific gene. Its protein level expression was detected by immunofluorescence, and with reference to changes in the marker of the mouse pluripotent stem cells, Oct4, to determine whether the mouse trophoblast stem cell lineage differentiation has occurred.

Through this experiment (Figure 3.3), the inventors found that mouse ciTotiSCs could have significant expression of CDX2 and down-regulated expression of Oct4 after induced differentiation, so they had the ability to differentiate into extra-embryonic trophoblast stem cells; while embryonic stem cells (mESCs) and potential expanded pluripotent stem cells (mEPS) could also have down-regulated Oct4 gene expression after induced differentiation, but they had almost no expression of CDX2, so they did not have the ability to differentiate into extra-embryonic trophoblast stem cells.

The inventors also found that totipotent stem cells (ciTotiSCs) of different passages (P1-P8) could gradually differentiate into pluripotent embryonic stem cells (rESC^{ciTotiSC}) after being changed into mESC media (2i/LIF), which was reflected in the down-regulation of totipotency genes and upregulation of pluripotency genes, thereby mimicking normal embryonic development (Figure 3.4)

Immunofluorescence staining steps:
Fixation: After the samples in the cell culture plate were washed with phosphate buffered saline (DPBS), the cells were fixed with 4% paraformaldehyde solution, kept at 4°C for 30 min, washed with DPBS for 4 times.
Blocking: A blocking solution (10% donkey serum + 1% BSA + 0.3% Triton-X100, diluted in DPBS) was added, blocked at room temperature for 1 h, washed with DPBS for 3 times.
Primary antibody incubation: The primary antibody was mouse anti-CDX2 (1:150, BioGenex, MU392A-UC). The antibody was diluted with DPBS containing 1% BSA according to the required concentration, incubated for 2 h at room temperature or overnight at 4°C, washed with DPBS for 5 min × 3 times.
Secondary antibody incubation: The secondary antibody was Donkey anti Mouse 555 (1:500, Life Technology, A-31570). The corresponding fluorescently labeled secondary antibody was diluted at 1:1000 in DPBS containing 1% BSA, incubated at room temperature for 1 h in the dark.
Nuclear staining: DAPI (nuclear dye, 4',6-diamidino-2-phenylindole dihydrochloride) to 1 µg/ml in DPBS, incubated for 5 min at room temperature;
Imaging: After being washed with DPBS for 5 min × 3 times, it was placed under an Olympus inverted fluorescence microscope IX83 for observation and photography.

### 3.2 Analysis of mouse trophoblast stem cell specific gene expression by RT-qPCR

Analysis of transcription of mouse trophoblast stem cell specific genes of mouse ciTotiSCs, embryonic stem cells (mESCs) and pluripotent expanded potential stem cells (mEPSCs) on days 0, 4, and 8 during induction into mouse trophoblast stem cells by RT-qPCR. The inventors found that mouse ciTotiSCs gradually activated trophoblast stem cell specific marker genes, including CDX2, Elf5, TFAP2C and Esx1, during the process of induced differentiation, which was similar to that of trophoblast stem cells (TSCs) as the positive control. In contrast, in embryonic stem cells (mESCs) and pluripotent expanded potential stem cells (mEPSCs), significant transcriptional increase was not detected in trophoblast stem cell specific marker genes during induction (Figure 3.2).

### Example 4: Comparison of the in vitro embryoid body differentiation and in vivo teratoma differentiation abilities of mouse ciTotiSCs and mouse embryonic stem cells (mESCs)

### 4.1 Comparison of embryoid body differentiation ability of mouse ciTotiSCs and mouse embryonic stem cells (mESCs) in vitro

After the mouse ciTotiSCs and mouse embryonic stem cells (mESCs) were digested with 0.05% trypsin, they were plated on 0.3% gelatin-coated cell culture plates. After half an hour, the suspension cells were collected, and the feeder cells were allowed to adhere to the cell culture plate to remove the feeder cells.

Feeder-depleted mouse ciTotiSCs or embryonic stem cells (mESCs) were resuspended in mouse embryoid body formation media (Knockout DMEM basal medium further supplemented with 10% fetal bovine serum (FBS), 1% GlutaMAX^{™} (L-glutamine substitute), 1% dual antibiotics (penicillin/streptomycin), 1% non-essential amino acids, 0.1 mM β-mercaptoethanol) at a density of 1 × 10⁵ cells per ml. Then, the embryoid body formation experiment was carried out by the hanging drop method: hanging drops were carried out on the lid of a 10 cm culture dish, and each 20 µl of the cell mixture was a hanging drop, and placed in a 5% CO₂, 37°C incubator for culturing. Two days later, the hanging drop cells were collected and placed in a 6-well low-adsorption culture plate for continued culturing, and embryoid bodies were harvested on days 0, 3, and 6 for identification using immunofluorescence staining.

Through immunofluorescence staining, the inventors found that CDX2-positive cells (representing extra-embryonic trophoblast cells, indicating that totipotent stem cells could differentiate into extra-embryonic cell types) could be detected in embryoid bodies derived from mouse ciTotiSCs on day 6, but no CDX2-positive cells (indicating that pluripotent stem cells were not differentiated to extra-embryonic cell types) (Figure 4.1) were detected in embryoid bodies derived from mouse embryonic stem cells (mESCs). By calculating the immunofluorescence staining results, the inventors found that CDX2-positive cells were detected in all embryoid bodies derived from mouse ciTotiSCs on days 3 and 6 (6/6 on day 3 and 17/17 on day 6), but no CDX2-positive cells were detected in embryoid bodies derived from mouse embryonic stem cells (mESCs) (0/7 on day 3 and 0/21 on day 6) (Figure 4.2).

CDX2 is a classical mouse trophoblast stem cell specific gene. Embryoid body differentiation experiment has demonstrated that mouse ciTotiSCs have the ability to differentiate into extra-embryonic trophoblast stem cells in vitro, which pluripotent stem cells do not have.

### 4.2 Comparison of in vivo teratoma differentiation ability of mouse ciTotiSCs and mouse embryonic stem cells (mESCs)

The teratoma assay is a classic assay to test the ability of cells to randomly differentiate into the three endodermal germ layers and extra-embryonic lineages.

Teratoma Differentiation Experimental Procedure:
After the mouse ciTotiSCs or mouse embryonic stem cells (mESCs) were digested under feeder cell culture conditions with 0.05% trypsin-EDTA into single cells, the cells were resuspended with mouse ciTotiSCs media or mouse embryonic stem cell (mESC) media, respectively, plated on 0.3% gelatin-coated cell culture plates, incubated in a 37°C incubator for 30 min to remove feeder cells.

The supernatant containing mouse ciTotiSCs or mouse embryonic stem cell (mESC) single cells was collected, and the cells were resuspended in DPBS.

The resuspended cells were injected subcutaneously into the groin of the hind legs of immunodeficient SCID mice, and the number of cells injected per mouse was about 1.0×10⁶.

Five weeks after the cells were subcutaneously injected, the mice were sacrificed by cervical dislocation, and the teratoma was removed from the subcutaneous tissue, fixed with 4% paraformaldehyde solution, and subjected to paraffin sectioning and HE staining.

The results of tissue sections were observed under a microscope for the tissue structures specific to the intraembryonic three germ layers and extra-embryonic cell lineages.

The inventors injected mouse ciTotiSCs or mouse embryonic stem cells (mESCs) subcutaneously into immunodeficient SCID mice, and both cell lines were able to form teratomas. After paraffin sectioning and HE staining of these teratomas, typical ectoderm (left column), mesoderm (middle column), and endoderm (right column) histology can be observed under the microscope. This indicated that mouse ciTotiSCs and mouse embryonic stem cells (mESCs) had the ability to differentiate into three germ layers in the embryo (Figure 4.3).

Next, the inventors further observed whether the HE-stained sections of teratomas formed by mouse ciTotiSCs or mouse embryonic stem cells (mESCs) have the classical extra-embryonic lineage tissue structure. It was found upon observation that in the teratomas formed by induced totipotent stem cells in mice, the classical extra-embryonic lineage-placental giant cells could be observed in the area rich in internal bleeding. The classical extra-embryonic lineage-placental giant cells exhibited typical morphological features with larger nuclei and larger cell volume, and expressed the placental giant cell marker gene PL-1 (Figure 4.4). In contrast, no extra-embryonic lineage was seen in teratomas formed from mouse embryonic stem cells (mESCs).

In vivo teratoma differentiation experiments proved that mouse ciTotiSCs had the ability to differentiate into extra-embryonic cells, while pluripotent stem cells did not have the ability.

### Example 5: In vivo chimeric assay of mouse ciTotiSCs (in vitro E4.5 embryonic stage)

### Schematic diagram of the chimeric assay process (Figure 5.1)

### Steps of chimeric assays:

Rosa26-tdTomato mouse blastocysts were used to establish mouse induced totipotent stem cell lines and mouse pluripotent embryonic stem cell lines (mESCs) labeled with red fluorescence, for 8-cell stage embryo injection. If the stem cells injected into the mouse 8-cell embryo developed with the embryo and were differentiated into the intra- or extra-embryonic part, red fluorescence (tdTomato) could be observed in the corresponding intra- or extra-embryonic part. The two cell lines were digested into single cells with 0.05% trypsin-EDTA, resuspended in the corresponding media, plated on 0.3% gelatin-coated cell culture plates, incubated for 30 min in a 37°C incubator to remove feeder cells. The cells were collected and resuspended in the corresponding media.

After superovulation, the purchased commercial ICR female mice were mated with commercial ICR male mice. After 1.5 days, 8-cell stage embryos were collected from the oviducts of successfully mated female mice. Chimeric embryos were obtained by injecting 5-10 mouse ciTotiSCs or mouse embryonic stem cells (mESCs) after depletion of feeder cells into each 8-cell stage embryo.

For mouse induced totipotent stem cell chimeric embryo E4.5 assay (Example 5, Figures 5.2, 5.3 and 5.4), after the injected 8-cell stage embryos were placed in KSOM media, and cultured in a 5% CO₂, 37°C incubator for 48 h, tdTomato-positive cells were analyzed for chimerism in the inner cell mass (ICM) and trophectoderm (TE).

For the experiments of in vivo development of mouse induced totipotent stem cell chimeric embryos to E7.5 and E12.5 (Examples 6-7, Figures 6-7), the injected 8-cell stage embryos were placed in KSOM media and recovered for culturing in a 5% CO₂, 37°C incubator for 1-2 h. They were then transplanted into the uterus of pseudopregnant ICR females 0.5 days after mating with ligated ICR males for further development.

**5.1** During the early embryonic development, the fertilized egg with totipotency gradually developed into the inner cell mass (ICM) in the embryo and the trophectoderm (TE) outside the embryo. Mouse ciTotiSCs and mouse embryonic stem cells (mESCs) for 8-cell injection had stable expression of red fluorescence (tdTomato). By analyzing the chimerism of tdTomato-positive cells, the inventors observed that mouse ciTotiSCs could be chimerized into both the inner cell mass (ICM) in the embryo and the trophectoderm (TE) outside the embryo. However, mouse embryonic stem cells (mESCs) could only be chimerized into the inner cell mass (ICM) of the embryo, but not the trophectoderm (TE) outside the embryo (Figure 5.2).

**5.2** The chimerism ratio of mouse ciTotiSCs and mouse embryonic stem cells (mESCs) in the inner cell mass (ICM) and trophectoderm (TE) was calculated (Figure 5.3).

Mouse ciTotiSCs: chimerized into both TE and ICM: 18/21 (85.7%); chimerized to TE only: 2/21 (9.5%); chimerized to ICM only: 1/21 (4.8%). That is, mouse ciTotiSCs had both intra-embryonic (inner cell mass) and extra-embryonic (trophectoderm) developmental potential.

Mouse Embryonic Stem Cells (mESCs): chimerized into both TE and ICM: 0/21 (0%); chimerized to TE only: 0/21 (0%); chimerized to ICM only: 21/21 (100%). That is, mouse pluripotent stem cells only had the ability to develop within the embryo (inner cell mass).

**5.3** CDX2 is a classic marker of the trophectoderm (TE). The inventors further confirmed with immunofluorescence staining of CDX2 whether tdtomato fluorescently labeled mouse ciTotiSCs chimerized into trophectoderm (TE) could express CDX2, a key marker of trophectoderm. The staining results showed (Figure 5.4) that tdtomato fluorescently labeled cells could express CDX2 at the same time. This further demonstrated that mouse ciTotiSCs were indeed involved in the development of trophectoderm (TE) in E4.5 day chimeric embryos, i.e., they had the ability to differentiate into extra-embryonic parts.

### Example 6: In vivo development of chimeric embryos of mouse induced totipotent stem cell to E7.5

7 days after transplantation of chimeric embryos from 8-cell stage embryos injected with mouse ciTotiSCs or mouse embryonic stem cells (mESCs) above into pseudopregnant mice, embryos that developed to E7.5 after the transplantation were isolated. Embryos at this stage (E7.5) comprised three parts: epiblast (EPI; after embryo implantation, EPI produced embryonic tissue containing three germ layers), placental cone (EPC), and extra-embryonic ectoderm (ExE). The experimental protocol is shown in Figure 5.1. By analyzing the chimerism of tdTomato-positive cells, immunofluorescence staining was performed for the classical marker of embryonic epiderm (EPI) OCT4, placental cone (EPC), and the classical marker of extra-embryonic ectoderm (ExE) (Figure 6.1). The inventors found that mouse ciTotiSCs could almost be chimerized into the entire E7.5 embryo, including intraembryonic epiblast (EPI), extra-embryonic placental cone (EPC) and extra-embryonic ectoderm (ExE). In addition, it was further found by immunofluorescence staining that tdTomato-positive cells derived from mouse ciTotiSCs could express the embryonic epiblast marker OCT4 and the extra-embryonic (embryonic cone EPC and extra-embryonic ectoderm EXE) marker ELF5. This experiment demonstrated that the mouse ciTotiSCs still had the ability to participate in the development of intra- and extra-embryonic tissues at E7.5 days after the mouse embryo implantation.

### Example 7: In vivo development of chimeric embryos of mouse induced totipotent stem cell to E12.5

Thirteen days after transplantation of chimeric embryos obtained by injecting mouse ciTotiSCs or mouse embryonic stem cells (mESCs) in 8-cell stage embryos, into pseudopregnant mice, they were developed into the embryo (Em), placenta (Pl) and yolk sac (Yo) of E12.5-E13.5 mice after the transplant was isolated. The experimental protocol is shown in Figure 5.1. By analyzing the chimerism of tdTomato-positive cells, the inventors found that mouse ciTotiSCs could be chimerized into mouse embryo (Em) and extra-embryonic tissue placenta (Pl) and yolk sac (Yo) in a high proportion (Figure 7.1). This experiment demonstrated that mouse ciTotiSCs still had the ability to participate in the development of embryos and extra-embryonic tissues (placenta and amniotic membrane) at E12.5-13.5 days after mouse embryo implantation.

To further analyze the proportion of tdTomato-positive cells derived from mouse ciTotiSCs to the chimerism of each part of the embryonic/extra-embryo, the inventors digested mouse embryo (Em), extra-embryonic tissue placenta (Pl) and yolk sac (Yo), respectively and analyzed chimeric ratio of mouse ciTotiSCs (tdTomato positive cells) in each tissue by flow cytometry..

### Steps of flow cytometry:

Before flow cytometry analysis of mouse embryo (Em), extra-embryonic tissue placenta (Pl) and yolk sac (Yo), they were washed twice with DPBS and cut into small pieces of about 1 mm with micromanipulator scissors. Mouse embryonic (Em) cells were digested with collagenase IV (1 U/ml of DNase added) for 30 min in a 37°C incubator, followed by digestion with TrypLE for 5 min; placental (Pl) cells were digested with accutase for 10 min in a 37°C incubator; Yolk sac (Yo) cells were digested with collagenase IV supplemented with 1 U/ml of DNase in a 37°C incubator for 5 min, followed by digestion with TrypLE for 3 min. The digestion reaction was terminated with 3 times the enzyme volume of DPBS+10% fetal bovine serum. After centrifugation at 800 rpm for 5 min, the cell pellet was resuspended in DPBS, the cells were filtered through a 70 µm cell mesh, and the single-cell filtrate was collected. The cell filtrate was transferred to flow assay tubes for analysis or sorting on the BD FACS Aria III as required by the experiment.

Data were analyzed using FlowJo v10 software. The analysis found that the in the uninjected group no tdTomato positive cells could be detected in mouse embryo (Em), extra-embryonic tissue placenta (Pl) and yolk sac (Yo), which served as a negative control; in the mouse embryonic stem cell (mESC) injection group, only tdTomato-positive cells could be detected in the mouse embryo (Em), while almost no tdTomato-positive cells could be detected in the extra-embryonic tissue placenta (Pl) and yolk sac (Yo); for the mouse induced totipotent stem cell injection group, a high proportion of tdTomato-positive cells could be detected in the mouse embryo (Em), extra-embryonic tissue placenta (Pl) and yolk sac (Yo). This also indicated that mouse ciTotiSCs could be efficiently chimerized into mouse embryo (Em), extra-embryonic tissue placenta (Pl) and yolk sac (Yo) (Figure 7.2).

The immunohistochemical analysis of the frozen section of the chimeric placenta showed that the presence of tdTomato positive cells could not be detected in the placenta in the uninjected group, which served as a negative control; in the mouse embryonic stem cell (mESC) injection group, only a few tdToamto positive cells were observed in the Laby area of the placenta (though this area was part of the extra-embryonic placenta, there were a small number of intraembryonic cells), and the fluorescent signal of tdTomato could not be co-localized with the fluorescent signals of placental extra-embryonic lineage cell markers CK8 and proliferin; in the mouse induced totipotent stem cell injection group, a high proportion of tdTomato-positive cells were observed in the extra-embryonic parts of the placenta (JZ and Laby regions), and the tdTomato fluorescence signal could be co-localized well with the fluorescence signals of placental extra-embryonic lineage cell markers CK8 and proliferin (Figure 7.3).

The immunohistochemical analysis of chimeric mouse embryos after frozen section found that both the mouse induced totipotent stem cell injection group and the mouse embryonic stem cell (mESC) injection group could be chimerized into various fetal tissues (including brain, heart and liver) in a high proportion, but the mouse induced totipotent stem cell injection group had higher chimerism efficiency (Figure 7.4).

### Example 8: Independent development of mouse ciTotiSCs into mouse embryos and living individuals

### 8.1 Single mouse induced totipotent stem cell (ciTotiSC) has bidirectional chimeric ability to intra- and extra-embryonic cells.

To more rigorously examine the developmental potential of totipotent stem cells (ciTotiSCs), the inventors injected single tdtomato-labeled mESCs or ciTotiSCs into mouse 8-cell embryos. After 48 hours of in vitro culture, the chimeric embryos developed to the late blastocyst stage (E4.5). As expected, mESCs were only chimerized into embryonic ICMs. In contrast, totipotent stem cells (ciTotiSCs) were chimerized into both ICM and TE. By further immunostaining the TE-specific marker CDX2, the inventors confirmed that a single totipotent stem cell (ciTotiSC) could indeed develop to the extra-embryonic TE lineage and correctly expressed the lineage marker CDX2 (Figure 8.1, top). In addition, the inventors transplanted 8-cell embryos injected with monopotent stem cells (ciTotiSCs) or mESCs into the fallopian tubes of pseudopregnant female mice, and further observed the chimera of E6.5-E7.5. The inventors observed that a single totipotent stem cell (ciTotiSC) could be chimerized into the intra-embryonic lineage (EPI) and extra-embryonic lineage (ExE and EPC), and this was confirmed by co-expression of tdTomato with OCT4 or ELF5. In contrast, mESCs were only chimerized into OCT4+ EPI (Figure 8.1, bottom). These data demonstrated that a single totipotent stem cell (ciTotiSC) possessed chimeric capacity for both intra- and extra-embryonic lineages, meeting the stringent totipotency criteria.

### 8.2 Induced totipotent stem cells (ciTotiSCs) could chimerically develop into a variety of cell types in and out of E13.5 embryos

To fully characterize the cell types developed from totipotent stem cells (ciTotiSCs) in extra-embryonic tissues before and after E13.5, the inventors used scRNA-seq to analyze tdTomato+ cells from totipotent stem cells (ciTotiSCs) in chimeric placenta and yolk sac. After alignment with specific genes of extra-embryonic lineages, the inventors confirmed that totipotent stem cell (ciTotiSC)-derived cells could develop into extra-embryonic trophoblast and yolk sac cell types, such as visceral yolk sac cells (Apoa4+ Fxyd2+ Entpd2+), sponge trophoblast cells (Tpbpa+ Rhox9+) and syncytiotrophoblast cells (Itm2a+), and cells of embryonic origin include erythrocytes, macrophages and monocytes (Figure 8.2).

### 8.3 Analysis of germline transmission ability of mouse ciTotiSCs

Totipotent stem cells (ciTotiSCs) have the ability to chimerize into the germinal ridge and to produce healthy chimeric offspring (Figure 8.3).

### 8.4 Independent development of mouse ciTotiSCs into induced mouse blastocysts in vitro

The mouse ciTotiSCs cultured under the condition of feeder cells were digested into single cells with 0.05% trypsin-EDTA, resuspend in a totipotent stem cell medium described in Example 1, transferred to a 6-well plate coated with 0.3% gelatin, and incubated in a 37°C incubator for 30 min to remove feeder cells. Mouse ciTotiSCs were collected, resuspended in the medium used to induce mouse blastocysts, and filtered through a 40 µm filter to remove impurities and undigested cell clumps. AggreWell 400 (STEMCELL Technologies, 34415) was pretreated according to the instructions. About 6,000 mouse ciTotiSCs were seeded in one well of a 6-well plate of AggreWell 400 (containing 1,200 chambers) and cultured in the medium for inducing mouse blastocysts. The induced mouse blastocysts could be obtained by centrifuging the AggreWell 400 culture plate at 100 g for 3 min to settle the cells, and then placing the culture plate in a 37°C incubator for 4-5 days. The inventors observed that the induced blastocysts had morphological characteristics highly similar to those of normal blastocysts (Figure 8.4). Statistics showed that the efficiency of induced blastocysts from mouse ciTotiSCs was about 70% (Figure 8.4).

Medium for inducing mouse blastocysts (Li, R. et al. Generation of Blastocyst-like Structures from Mouse Embryonic and Adult Cell Cultures. Cell 179, 687-702 e618, doi:10.1016/j.cell.2019.09.029 (2019)): 25% trophectoderm stem cell basal medium (RPMI 1640 basal medium supplemented with 20% fetal bovine serum (FBS), 1X GlutaMAX (L-glutamine substitute), 1X sodium pyruvate (Gibco, 11360070), and 0.1 mM 2-mercaptoethanol), 25%N2B27 basal medium (1:1 mixture of DMEM/F-12 and Neurobasal 1:1 as basal medium, add 0.5 × N2 (17502-048), 0.5X B27 (17504-044), 1X nonessential amino acids), 1X GlutaMAX (L-glutamine substitute), 0.1 mM 2-mercaptoethanol, 0.1% BSA or 5% KSR), and 50% KSOM (NaCl (95 mM), KCl (2.5 mM), KH2PO4 (0.35 mM), MgSO4 (0.2 mM), NaHCO3 (25 mM), CaCl2 (1.71 mM), Na2-EDTA (0.01 mM), L-glutamine (1.0 mM), sodium lactate (10 mM), sodium pyruvate (0.2 mM), glucose (5.56 mM), essential amino acids (EAA; 10.0 mL/l), non-essential amino acids (NEAA; 5.0 mL/l), BSA (4 g/l).). The following were additionally added: 2 mM Y-27632 (only added on day 1), 12.5 ng/mL rhFGF4 (R&D, 235F4025), 0.5 mg/mL Heparin (Sigma-Aldrich, H3149), 3 mM GSK3 inhibitor CHIR99021 (Reagents Direct, 27-H76), 5 ng/mL BMP4 (Proteintech, HZ-1040), and 0.5 mMA83-01.

### 8.5 Mouse blastocysts induced by mouse ciTotiSCs possess three cell lineages of normal mouse blastocysts.

The normal mouse late blastocyst (E4.5) mainly contains three cell lineages: inner cell mass (ICM, specific expressing OCT4), trophectoderm (TE, specific expressing CDX2), and primitive endoderm (PrE, specific expressing SOX17).

In order to detect whether the induced blastocysts derived from mouse ciTotiSCs have the three cell lineages of normal mouse blastocysts, the inventors performed immunofluorescence staining on the blastocysts induced for 4 days. It was found by staining that the induced blastocysts could express the inner cell mass marker OCT4, the trophectoderm marker CDX2 and the primitive endoderm marker SOX17, and the expression pattern was consistent with that of normal mouse blastocysts. Statistics showed that the proportion of induced blastocysts that correctly expressed the three cell markers was about 82% (Figure 8.5).

Therefore, these results demonstrated that mouse ciTotiSCs could be efficiently induced into mouse blastocysts with the correct structure and gene expression pattern.

### 8.6 Induced blastocysts derived from mouse ciTotiSCs can be developed post-implantation in vitro

The inventors further cultured the induced blastocysts derived from mouse ciTotiSCs using an in vitro embryo culture system, and found that the induced blastocysts could be cultured in vitro to produce a cylindrical structure. Ectoderm (stained positive for TFAP2C) and EPI (stained positive for Oct4) as two hemispheres were surrounded by endoderm (stained positive for SOX17), which was similar to postimplantation embryos in mouse E4.5-E5.5 stage (Figure 8.6).

The specific culturing method was as follows: Induced blastocysts were picked with a mouth pipette, washed twice in an IVC-1 medium (Cell Guidance Systems, M11), and then transferred to a u-Slide 8-well plate (ibidi, 80826) supplemented with IVC-1 medium. Approximately 20-30 induced blastocysts were placed in one well of a u-Slide 8-well plate. After induction of blastocyst adherence, the medium was changed to IVC-2 (Cell Guidance Systems, M12). About 2-4 days later, the post-implantation embryo-like structure appeared, which was fixed with 4% PFA at room temperature for 15 min, and then the next step, analysis by immunofluorescence staining, was conducted.

### 8.7 In vivo implantation of induced blastocysts derived from mouse ciTotiSCs into mouse uterus for further development

Currently, the well-accepted standard for verifying that blastocysts are truly functional is to transfer blastocysts obtained in vitro into the uterus of pseudopregnant mice to see if they can be implanted and develop into a fetus. To this end, the inventors transplanted induced blastocysts into the uterus of 2.5-day-old pseudopregnant mice. At 7.5 dpc, decidual implantation sites could be formed in the uterus of the induced blastocysts. The size of the decidua produced by the induced blastocysts varied, but most of them were similar to normal mouse decidua, and some were slightly smaller. Since the initial mouse ciTotiSCs carried the tdTomato red fluorescent marker, further dissection of the decidual tissue revealed that the embryonic structures in the decidua were positive for tdTomato, demonstrating that the structure was formed by the implantation and further development of induced blastocysts derived from mouse ciTotiSCs in utero (Figure 8.7). These results demonstrated that induced blastocysts derived from mouse ciTotiSCs could be implanted in the uterus to trigger decidualization and further develop into a certain embryonic structure.

### Example 9: Effects of various small molecule reagents on gene expression

By analyzing the expression of specific gene sets by RNA-seq data, it is very clear that the inventors found that TTNPB, 1-AKP, WS6 and mLIF were all important for the induction of totipotent stem cells, which was reflected in the significant upregulation of totipotency genes, maternal genes and ZGA genes (Figure 10a).

Among the three compounds, the RA agonist TTNPB could directly activate a large number of totipotency, maternal and ZGA genes rich in RAR-binding motifs (Figures 10d-g). TTNPB or Trans-RA, another widely used RA agonist, exhibited similar induction effects on totipotency genes, further confirming the specificity of the RA signaling pathway in the induction of totipotent cells; whereas in the presence of the RA antagonist AGN193109, the pluripotency gene could not be induced (Figures 10e,f). These results confirmed the central role of the retinoic acid signaling pathway in establishing totipotent in cells.

1-Azakenpaullone (1-AKP) is a selective dual inhibitor of GSK3β and CDK1/cyclin B. Studies have shown that 1-AKP treatment induces specific Wnt signaling downstream gene expression and G2/M arrest simultaneously. GO analysis consistently showed that specific Wnt signaling-targeted genes were not up-regulated after 1-AKP was withdrawn in totipotent stem cell (ciTotiSC) culture conditions (Figure 10h). Meanwhile, the inventors also observed a prolongation of the G2 phase of the cell cycle after induction of totipotent stem cells (ciTotiSCs), which was consistent with the difference between early 2C embryos and blastocysts (Figure 10i). G2 phase prolongation of totipotent stem cells (ciTotiSCs) was reduced after 1-Azakenpaullone was withdrawn from totipotent stem cell (ciTotiSC) culture conditions, suggesting that 1-Azakenpaullone maintained, to a certain extent, a feature associated with poikilogenesis in totipotent stem cells (ciTotiSCs)-prolongation of G2 phase (Figure 10i). These results suggested that the mechanism by which 1-Azakenpaullone promoted induction of totipotency was at least in part through specific Wnt signaling activation and cell cycle G2 phase prolongation.

WS6 is an IKK-NF-κB inhibitor that was previously shown to promote post-mitotic cell proliferation. GO analysis of differential genes after WS6 ablation in totipotent stem cell (ciTotiSC) culture conditions enriched multiple innate immunity-related pathways, especially NF-κB signaling (Figure 10j). Interestingly, some previous studies have shown that expression of endogenous retrovirus (ERV) can trigger NF-κB signaling through TLR or cGAS recognition of ERV-derived cytoplasmic double-stranded RNA (dsRNA) or double-stranded DNA (dsDNA) (Chiappinelli *et al.* 2015; Lima-Junior *et al.* 2021; Mao *et al.* 2022). Consistently, the expression of genes downstream of NF-κB signaling is also up-regulated when WS6 is withdrawn (Figure 10k). Furthermore, it has been reported that downregulation of NF-κB during zygote development is critical for its totipotent state, and activation of NF-κB triggers subsequent development after ZGA (Akihiko Nishikimi, 1999; Paciolla *et al.* 2011). Therefore, WS6 may play a role in promoting and stabilizing induction of totipotent stem cells (ciTotiSCs), in part by inhibiting the NF-κB mediated immune response triggered by ERV activation (Figures 10k, j).

## Claims

1. A composition comprising:
(a) a RA signaling pathway activator; and
(b) one or more of a GSK-3 inhibitor, an IKK signaling pathway inhibitor, an HDAC inhibitor, a histone methyltransferase inhibitor, a Src kinase inhibitor, a cAMP activator, and a cell metabolism modulator.

2. A kit comprising:
(a) a RA signaling pathway activator; and
(b) one or more of a GSK-3 inhibitor, an IKK signaling pathway inhibitor, an HDAC inhibitor, a histone methyltransferase inhibitor, a Src kinase inhibitor, a cAMP activator, and a cell metabolism modulator.

3. Use of (a) a RA signaling pathway activator and (b) one or more of a GSK-3 inhibitor, an IKK signaling pathway inhibitor, an HDAC inhibitor, a histone methyltransferase inhibitor, a Src kinase inhibitor, a cAMP activator, and a cell metabolism modulator in manufacturing induced totipotent stem cells.

4. The composition, kit or use according to any of the preceding claims, wherein the RA signaling pathway activator is selected from small molecules for the same pathway such as TTNPB, Tretionin/RA/ATRA, AM580, Taza, 9-cis-RA, Acitretin, CD437, Tamibarotene, Tazarotene, Retinoic acid, Isotretinoin, Acitretin sodium, ch55 and AC55649, and the others.

5. The composition, kit or use according to any of the preceding claims, wherein the GSK-3 inhibitor is selected from small molecules for the same pathway such as 1-Azakenpaullone, AZD2858, CHIR99021, AZD1080, and the others.

6. The composition, kit or use according to any of the preceding claims, wherein the IKK signaling pathway inhibitor is selected from the small molecules for the same pathway such as WS6, sc-514, PF184 and IKK16, and the others.

7. The composition, kit or use according to any of the preceding claims, wherein the HDAC inhibitor is selected from small molecules for the same pathway such as Trichostatin A (TSA), Valproic acid (VPA), Vorinostat (SAHA) and Entinostat (MS-275), and the others.

8. The composition, kit or use according to any of the preceding claims, wherein the histone methyltransferase inhibitor is selected from small molecules for the same pathway such as BIX 01294, 3-deazaneplanocin A (DZNeP) HCl, A-366, UNC0638 and SGC 0946, and the others.

9. The composition, kit or use according to any of the preceding claims, wherein the Src kinase inhibitor is selected from small molecules for the same pathway such as Dasatinib (BMS-354825), WH-4-023, Ponatinib (AP24534), Bosutinib (SKI-606), and the others.

10. The composition, kit or use according to any of the preceding claims, wherein the cAMP activator is selected from small molecules for the same pathway such as Colforsin (Forskolin, HL 362) and 8-Br-cAMP, and the others.

11. The composition, kit or use according to any of the preceding claims, wherein the cell metabolism modulator is selected from the cell metabolism modulators such as 2-Deoxy-D-glucose (2-DG), sodium acetate, sodium L-lactate and D-ribose, and the others.

12. A culture medium comprising the composition according to any of the preceding claims.

13. The culture medium according to any of the preceding claims, wherein the culture medium comprises a basal culture medium.

14. The culture medium according to any of the preceding claims, wherein the basal culture medium is selected from common basal culture media such as DMEM, Knockout DMEM, RPMI 1640 and DMEM/F12, and the others.

15. A method of manufacturing induced totipotent stem cells, comprising culturing pluripotent stem cells in the culture medium according to any of the preceding claims, thereby manufacturing the induced totipotent stem cells.

16. The method according to any of the preceding claims, wherein the pluripotent stem cells are embryonic stem cells or induced pluripotent stem cells.

17. The method according to any of the preceding claims, comprising reprogramming non-pluripotent cells into pluripotent stem cells.

18. The method according to any of the preceding claims, wherein the non-pluripotent cells are selected from somatic cells and/or adult stem cells.

19. The method according to any of the preceding claims, wherein the reprogramming non-pluripotent cells into pluripotent stem cells comprises expressing one or more reprogramming factors selected from the group consisting of Oct4, Sox2, Klf4 and c-Myc in the non-pluripotent cells.

20. The method according to any of the preceding claims, wherein the culturing pluripotent stem cells is performed for 1, 2, 3, 4, 5, 6, 7, 8, 9, 10, 11, 12, 13, 14, 15, 16, 17, 18, 19 or 20 days.

21. A culture comprising the culture medium according to any of the preceding claims and pluripotent stem cells.

22. The culture according to any of the preceding claims, wherein the pluripotent stem cells are embryonic stem cells or induced pluripotent stem cells.

23. A culture comprising the culture medium according to any of the preceding claims and totipotent stem cells.

24. The culture according to any of the preceding claims, wherein the totipotent stem cells are induced totipotent stem cells, preferably manufactured by the method according to any of the preceding claims.

25. An induced totipotent stem cell, **characterized by** one or more of the following:
(a) increased transcription of one or more totipotent transcription markers selected from the group consisting of MERVL, Zscan4c, Zscan4d, Zscan4f, Zfp352, Tcstv1, Tcstv3, Teme92, Gm6763;
(b) reduced transcription of one or more pluripotent transcription markers selected from the group consisting of POU5f1, ZFP42, NANOG, KLF4, ESRRB; and
(c) the ability to differentiate into extra-embryonic cell type(s);
preferably the induced totipotent stem cells can be manufactured by the method according to any of the preceding claims.

26. An induced totipotent stem cell, which can be produced by the method according to any of the preceding claims.

27. An organism derived from the induced totipotent stem cell according to any of the preceding claims, preferably wherein the organism is a rodent animal or a mammal animal.

28. An organoid produced from the induced totipotent stem cell according to any of the preceding claims.

29. A tissue produced from the induced totipotent stem cell according to any of the preceding claims, preferably the tissue is blood.

30. A differentiated cell differentiated from the induced totipotent stem cell according to any of the preceding claims, preferably wherein the differentiated cell is a blood cell or an immune cell, such as a T cell or an NK cell.
